# DEMANDE DE BREVET EUROPEEN

(11) **EP 2 213 729 A2**
(43) Date de publication de la demande: **04.08.2010**
(21) Numéro de dépôt: 09168525.5
(22) Date de dépôt: 27.10.2004
(51) Int. Cl.: C12N 5/077, A61L 27/38, A61N 1/362, A61P 9/00, C12N 13/00

(54) **Stimulateur cardiaque orthorythmique inotrope**

(30) Priorité: 06.11.2003 FR 0313055; 20.02.2004 FR 0401736
(62) Demande divisionnaire de: 04805324.3
(71) Demandeur: Zacouto, Fred, 75015 Paris (FR)
(72) Inventeur: Zacouto, Fred, 75015 Paris (FR)
(74) Mandataire: Jacobson, Claude

(57) **Abrégé**

Dispositif de stimulation cardiaque automatique, programmable et implantable (STIO) permettant un contrôle du coeur, accompagné d'une forte augmentation de la contractilité des cellules myocardiques à chaque battement provoquée par un effet de renforcement post-extrasystolique optimalisé. La STIO ne cause pas de fatigue durable au myocarde, elle augmente fortement, instantanément et durablement le débit coronaire, fait régresser les dilatations des parois, s'oppose aux thromboses, aux arythmies. La STIO crée une involution génétique du processus pathologique soit par le seul effet de la mécanosensibilité de certaines expressions génétiques, soit par l'ajout d'une dédifférenciation cellulaire autologue partielle, obtenue par manipulation génétique originale, induisant une régénération physiologique et anatomique. Cette méthode permet également de créer des stents artériels vivants physiologiques et auto-contractiles, notamment coronaires, ainsi que la greffe de cellules myocardiques dédifférenciées.

## Description

L'invention a pour objet un dispositif de stimulation électrique du coeur permettant, notamment, d'améliorer les performances hémodynamiques, celles des cellules du coeur et en particulier le débit sanguin, et ceci notamment chez les patients souffrant d'une insuffisance cardiaque, ou d'une tachycardie ou arythmie, et notamment d'une insuffisance cardiaque sévère ou aiguë, gauche ou droite, systolique ou diastolique ou globale.

L'invention a également pour objet un procédé de pilotage d'un tel dispositif.

L'invention a encore pour objet un procédé de stimulation électrique du coeur ainsi que l'amélioration cellulaire du coeur mettant en oeuvre un tel dispositif, notamment chez le patient souffrant d'une insuffisance cardiaque, ou d'une tachycardie ou arythmie, et notamment d'une insuffisance cardiaque aiguë, gauche, droite, ou globale.

Jusqu'à présent, les dispositifs de stimulation automatique du coeur, appelés également pacemakers, sont destinés essentiellement à remplacer ou réguler l'électrogénèse spontanée d'activation du muscle cardiaque.

Un pas important a été franchi pour le contrôle et la réduction des tachycardies, tachyarythmies ou extrasystoles isolées dans les procédés et dispositifs décrits dans les brevets Zacouto US-A-4,052,991 et US-A-3,857,399. Ces perfectionnements permettaient, notamment, de modifier les intervalles de couplage de la stimulation électrique en fonction de la durée variable du ou des cycles antérieurs, par exemple selon un pourcentage de cette durée. Ces perfectionnements permettaient, en outre, d'envoyer des stimulations en salve et en rampes. Ils permettaient également des marquages différentiels des spots de détection et de stimulation locales sur l'électrocardiogramme.

La stimulation pairée (STP) ou couplée (STC) et inotrope optimale (STIO) essaie de provoquer manuellement ou automatiquement une succession périodique d'inhibitions et de stimulations myocardiques avec, de préférence, des paramètres permettant d'obtenir une hémodynamique maximale et une protection anti-arythmique. La STIO permet, en principe, une mobilisation plus forte des réserves et acquisitions énergétiques du myocarde généralement d'autant plus grande que celui-ci est davantage fatigué (Wayne Cooper : Postextrasystolic Potentialisation, Circulation, vol 88, n° 6, 2962, Dec 1993) telle que, par exemple, l'activation augmentée d'un cycle des pentoses en plus du cycle de Krebs (hexoses) et/ou une activation renforcée et resynchronisée de certains canaux ioniques et d'électrons transmembranaires, l'augmentation des concentrations et libérations intracellulaires d'ions de calcium, sécrétions de peptides vaso- ou myo- actifs et modifications de certaines expressions, traductions et transductions génétiques locales d'adaptations à une énergétique myocardique biochimique renforcée de type « coeur sportif ». Ce renforcement instantané, important et durable, sans limite connue dans le temps, de la contractilité après certaines extrasystoles est bien connu des cardiologues sous le nom de renforcement post-extrasystolique (RPE). Le RPE (ou PESP) est un phénomène naturel fréquent qui démontre la possibilité de mobiliser davantage l'acquisition et les dépenses des réserves énergétiques myocardiques, lesquelles restent souvent inhibées lors de ses défaillances (autres que certaines tachycardies ectopiques). La persistance du RPE même en cas de défaillance grave aiguë du myocarde (exemple : oedème pulmonaire après infarctus récent antérieur étendu du myocarde) ou chronique (Classificat. New-York Heart Assoc., classes 2 à 4) montre la possibilité de l'utilisation de la STP dans ces cas cliniques.

Cependant, la stimulation pairée (STP) publiée notamment depuis 1964, largement expérimentée chez l'animal et dans plusieurs essais cliniques pour réduire ou mieux supporter certaines tachycardies et défaillances myocardiques (par exemple : F. Zacouto et coll., Paris, Nouv. Presse Méd. 1974, 3, No 22, p. 1448), n'est pratiquement toujours pas utilisée en cardiologie. Ceci s'explique par l'usage d'équipements de stimulation et de détection du coeur tragiquement insuffisants car incapables de cibler en continu les zones critiques étroites et souvent instables dans chaque cycle cardiaque (CC) qu'il est pourtant indispensable d'atteindre pour réaliser une STP efficace optimale, continue, économe en oxygène et anti-arythmique.

D'une façon générale, les dispositifs actuellement utilisés ne permettent pas, par la stimulation électrique, d'améliorer durablement les performances hémodynamiques du muscle cardiaque, ceci notamment chez les patients où cette performance est significativement abaissée, notamment chez les insuffisants cardiaques graves.

On avait déjà observé depuis le XIXe siècle le phénomène de renforcement de la contraction post-extrasystolique (Post-extrasystololic PESP Potentiation en langue anglaise).

Ainsi, le déposant avait pu obtenir, chez deux patients en insuffisance ventriculaire gauche aiguë, une amélioration temporaire de l'hémodynamique en parvenant à coupler, après un réglage manuel de démarrage, une stimulation pairée comprenant des salves d'impulsions de stimulations consécutives rapprochées dans un même cycle cardiaque couplée, sur l'onde R, vers la fin de la zone réfractaire (F Zacouto et al. ; Paris, Nouv. Presse Méd., 1974, 3, No 22, p.1448).

Des recherches importantes ont été ensuite conduites sur le phénomène du renforcement post-extrasystolique dans le but d'essayer de profiter de l'amélioration de la contraction mécanique déclenchée par l'envoi de stimulations électriques provoquant une extrasystole stimulée (voir, par exemple, les documents US 3,939,844 et US 5,213,098). Cependant, malgré un travail important sur une longue durée, les chercheurs sont finalement parvenus à la conclusion que le problème consistant à établir, d'une façon durable et efficace, un renforcement de la contractilité myocardique par la stimulation pairée, n'a pas pu être résolu, et, malgré les promesses de ce principe, les résultats des études ont été décourageants (voir par exemple N. Wayne Cooper, Post-extrasystolic Potentialisation, Circulation, vol 88, No 6, 2962, Dec. 1993).

Ces échecs peuvent s'expliquer, notamment, par le fait que les zones fonctionnelles réfractaires électriques ZRE et myocardiques ZRM et les réactions métaboliques correspondantes peuvent varier significativement à chaque cycle, notamment en cas de souffrance du myocarde ou de troubles du rythme irrégulier.

Plus récemment, des propositions ont été faites pour créer des appareils capables d'adresser au coeur des salves pairées ou couplées d'impulsions destinées soit à provoquer, selon une hypothèse, une action de sensibilisation du myocarde pendant la durée réfractaire électrique, soit à générer, peu après la fin de la période réfractaire électrique, une électrosystole destinée à provoquer un renforcement post-extrasystolique lors d'une systole postérieure. Différents documents traitent de la recherche de ce renforcement tels que WO 02/53026 et WO 03/20364.

Cependant, ces recherches se heurtent au fait que la stimulation pairée ou couplée n'est pas compatible avec la présence, fréquente notamment chez les insuffisants cardiaques, d'arythmies ou tachyarythmies, quand elle ne les provoque pas elle-même sur des coeurs déjà instables.

La présente invention se propose, et ceci à l'encontre des difficultés et des conclusions décourageantes de la recherche, de fournir un dispositif et des procédés permettant une amélioration durable et significative de la performance hémodynamique du coeur par stimulation électrique.

Un autre objectif de l'invention est de traiter les arythmies ou les tachycardies, y compris les tachycardies récurrentes ou non rapidement réductibles par les pacemakers antitachycardiques (Zacouto, US-3 857 399 et US-4 052 991), y compris chez les insuffisants cardiaques et d'améliorer instantanément l'hémodynamique, que la tachycardie soit réduite ou non.

Dans son cadre général, l'invention se propose de perfectionner un dispositif de stimulation du muscle cardiaque permettant un accroissement significatif de la performance hémodynamique du coeur et/ou le traitement des tachycardies comprenant un dispositif de préférence implanté à demeure comprenant :
- des moyens d'acquisition automatique du rythme cardiaque, permettant notamment de connaître l'intervalle entre au moins les deux dernières ondes R (provoquées ou spontanées) du cycle cardiaque qui vient de s'achever,
- des moyens permettant de déterminer, de préférence en temps réel, la durée de la période réfractaire électrique (ZRE) qui suit la dernière onde R dudit cycle,
- et des moyens pour adresser, sensiblement sans délai vers ou à la fin de ladite période réfractaire (ZRE), au moins une impulsion de stimulation.

L'impulsion de stimulation de couplage ou celle des impulsions de la salve qui stimule le coeur génère une onde (R') n'entraînant aucune réaction mécanique, mais provoquant une zone réfractaire électrique supplémentaire ZR, contribuant à l'effet inotrope et anti-arythmique recherché.

Un objet de l'invention est un dispositif de stimulation et/ou renforcement du muscle cardiaque et/ou des cellules myocardiques permettant un accroissement significatif de la performance hémodynamique du coeur et/ou le traitement des tachycardies, tachyarythmies ou fibrillations auriculaires, comprenant :
- des moyens d'acquisition automatique du rythme cardiaque, et optionnellement de son origine, permettant notamment de connaître l'intervalle entre au moins les deux dernières ondes R (provoquées ou spontanées) du cycle cardiaque qui vient de s'achever,
- des moyens d'acquisition précise de l'hémodynamique cardiaque,
- des moyens permettant de déterminer, continuellement en temps réel la durée de la période réfractaire électrique (ZRE) qui suit la dernière onde R dudit cycle,
- des moyens permettant d'évaluer au moins un paramètre relatif à l'état fonctionnel cellulaire du myocarde,
- et des moyens subordonnés auxdits moyens d'évaluation pour adresser sensiblement sans délai à la fin de ladite période réfractaire (ZRE), au moins une impulsion de stimulation, pairée ou couplée, adaptée audit état fonctionnel cellulaire.

De façon particulièrement préférée, lesdits moyens d'évaluation déterminent une zone critique efficace à cibler ZCE, qui se place immédiatement après la fin de la zone réfractaire électrique, et se termine à la fin d'une zone réfractaire de contraction myocardique maximale ZRMM, l'envoi de ladite impulsion de stimulation intervenant dans ladite zone ZCE, si elle est présente et ciblable, et ne provoque pas d'arythmie intraitable. Dans ce dernier cas, des moyens pour reculer automatiquement, par exemple, de 20 ms en 20 ms, la zone ZCE sont prévus, tout en surveillant l'hémodynamique et les consommations métaboliques du myocarde.

De préférence, lesdits moyens d'évaluation essaient de détecter ou vérifier à chaque cycle le seuil d'excitabilité du myocarde.

De préférence, conformément à l'invention, le dispositif est agencé pour déterminer à chaque cycle, et de préférence en temps réel, la durée de ladite période réfractaire électrique (ZRE).

De façon avantageuse, la détermination de la zone réfractaire s'effectue dans une partie du coeur dans laquelle l'impulsion ou la salve de stimulation est adressée, par exemple en utilisant les mêmes électrodes pour la détection et la stimulation, ou des électrodes très voisines.

Dans une forme de réalisation avantageuse, on adresse, sensiblement vers la fin de ladite période réfractaire (ZRE), une salve d'impulsions de stimulation, la durée de la salve et l'intervalle de répétition desdites impulsions étant tels qu'une impulsion de stimulation de la salve se trouve adressée au coeur sensiblement sans délai après la fin de ladite période réfractaire.

De préférence, l'impulsion stimulante adressée vers ou à la fin de la période réfractaire est adressée, presque sans délai, par exemple entre 10 et 20 ms après la fin de la zone réfractaire ZRE. Dans le cas d'une salve, il est cependant nécessaire que le début de la salve se produise à l'intérieur et vers la fin de la période réfractaire.

On constate que l'on peut ainsi obtenir, par un simple dispositif implanté, un renforcement de la contractilité et de l'hémodynamique générée par l'une au moins des impulsions de stimulation de la salve, notamment en cas d'insuffisance cardiaque aiguë ou sévère.

Parallèlement, on obtient sur les coeurs tachycardiques une diminution permanente importante du rythme ventriculaire et un renforcement de la contractilité du coeur.

Il en résulte, notamment, que l'on obtient des contractions cardiaques significativement plus performantes et ceci sans entraîner une surconsommation en oxygène du coeur intolérable car l'augmentation de consommation d'oxygène se trouve compensée par l'augmentation concomitante des débits coronaires et surcompensée par le gain de la contractilité.

Le dispositif est ainsi capable d'effectuer une stimulation pairée ou couplée inotrope optimale, appelée ci-après STIO.

La détermination de la durée de la zone réfractaire ZRE du cycle en cours peut être menée à partir de caractéristiques du cycle précédent, ou même de cycles antérieurs. Par exemple, on peut l'évaluer *a priori* comme un pourcentage de la durée du cycle antérieur, ce pourcentage augmentant si la durée du cycle diminue. Par exemple, le pourcentage sera de 25 à 30 % pour un rythme de 60 p/mn et de 50 à 80 % pour un rythme de 120 p/mn.

L'évaluation de la durée de la zone réfractaire peut être effectuée par tout autre moyen déjà connu des cardiologues, par exemple par balayage progressif d'une impulsion électrique dans une salve retardée, de cycle en cycle jusqu'à l'acquisition d'une électrosystole générée. La durée ZRE obtenue peut alors être utilisée lors d'un ou lors des cycles suivants pour l'envoi de l'impulsion ou de la salve d'impulsions de stimulation.

Dans le cas où, au lieu d'une salve, le dispositif génère une seule impulsion de stimulation, les moyens (4) de détermination de la durée de la zone réfractaire sont agencés pour acquérir par un balayage d'une deuxième impulsion, la durée sensiblement exacte de la zone réfractaire, ce balayage étant mené par exemple durant les cycles cardiaques précédents ou en cours.

En cas d'utilisation d'une salve d'impulsions de stimulation, celle-ci débutera, de préférence, un peu avant la fin estimée de la période réfractaire ZRE et la durée de cette salve et, par conséquent, le nombre d'impulsions de stimulation sera avantageusement tel qu'une impulsion de stimulation pourra intervenir très vite après la fin de ladite période réfractaire.

Ces moyens de déterminer la durée d'une zone réfractaire du coeur sont connus des cardiologues.

Dans une variante de l'invention, dans le cas où la stimulation à la fin de la période réfractaire ZRE s'effectue par envoi d'une salve d'impulsions, le dispositif peut envoyer la salve sensiblement avant la fin estimée de la période réfractaire ZRE, et détecter sans délai, dans le même cycle, de préférence au même emplacement du coeur, laquelle des impulsions de la salve provoque une électrosystole R', pour une amplitude stimulante donnée, ce qui fournit la durée ZRE qui vient de s'écouler. Cette durée détectée permet, en outre, de déterminer la durée probable de la zone réfractaire ZRE du cycle suivant.

De préférence, il est prévu des moyens de stimulation anti-tachycardique et des moyens sensibles aux extrasystoles pour stopper automatiquement ladite stimulation en cas de survenue d'une instabilité hémodynamique trop grande ou d'arythmie électrique répondant à des critères prédéterminés.

Dans une forme de réalisation perfectionnée, on peut affiner la détermination de la zone réfractaire ZRE du cycle à venir en tenant compte d'un ou plusieurs paramètres détectés et ceci en tenant compte de la stabilité fonctionnelle et rythmique du coeur.

Ces paramètres peuvent être notamment :
- durée du ou des cycles antérieurs ou en cours,
- paramètres hémodynamiques, consommation métabolique et seuil d'excitabilité pendant un certain nombre de contractions consécutives comparables, par comparaison avec des seuils.

En cas de discordance importante entre, par exemple, la zone ZRE calculée en utilisant les rapports entre les intervalles QT et les intervalles RR réels du patient, comme cela est connu en cardiologie, et la zone ZRE mesurée, le myocarde peut subir une modification métabolique pouvant soit précéder une arythmie grave si la ZRE diminue, soit un rétablissement fonctionnel si la ZRE se rallonge.

L'appareil monitorise et différencie ces deux cas et réagit instantanément selon une programmation. Par exemple, en cas d'arythmie grave, on arrête la stimulation ou on modifie les paramètres de stimulation.

On peut prévoir que la salve d'impulsions de stimulation pourra être avancée ou reculée par rapport à une estimation de la zone réfractaire, et/ou l'intervalle entre les impulsions dans la salve et/ou l'amplitude des impulsions diminuée ou augmentée, le dispositif présentant des moyens d'acquisition automatiques, notamment par obtention de l'ECG intracardiaque permettant de déterminer quelle est l'impulsion de stimulation dans la salve qui a déclenché l'onde R', et, en conséquence, éventuellement modifier la salve.

Un tel dispositif sera particulièrement indiqué pour réduire les tachycardies, y compris les tachycardies sinusales des insuffisants cardiaques.

De préférence, le dispositif comporte des moyens sensibles aux ondes R spontanées ou stimulées et/ou à la détermination des zones réfractaires électriques et mécaniques, notamment par balayage de toute la salve ou seulement à l'intérieur de cette salve et/ou des moyens de détermination sans délai des seuils d'excitabilité du coeur, par exemple en prévoyant des impulsions de stimulation d'intensité variable, y compris infraliminaires pour permettre leur mesure. Il est aussi prévu de pouvoir commander le stimulateur comme un stimulateur orthorythmique classique anti-tachycardique et réagissant aux extrasystoles et de pouvoir stopper automatiquement la STIO programmée en cas de survenue d'une instabilité hémodynamique trop grande. En cas d'une instabilité trop élevée des zones réfractaires ou des seuils d'excitabilité du myocarde ou des deux simultanément, on peut, par exemple, augmenter les durées et/ou le nombre et/ou le voltage des impulsions des salves ou programmer l'arrêt du fonctionnement à partir d'un seuil.

De préférence, le dispositif se caractérise par le fait qu'il comprend, en outre, des moyens d'acquisition très précis de l'hémodynamique cardiaque.

Ces moyens sont, en soi, connus et comportent, de préférence, un ou des capteurs intracardiaques de pression ou de tels capteurs disposés à proximité. De préférence, des capteurs pour la détermination des variations de volume cardiaque, par exemple des capteurs de proximité d'impédance électrique connus, notamment juxta- et intracardiaques, sont également utilisés, afin d'acquérir les courbes précises pression/volume de la contraction cardiaque.

Dans une forme de réalisation perfectionnée de l'invention, un dispositif selon l'invention, implantable ou même externe non implantable, peut comprendre, outre les moyens d'acquisition de rythme, les moyens de détermination de durée de zone réfractaire et les moyens d'envoi d'une impulsion ou d'une salve, tels que décrits ci-dessus, des moyens sensibles à l'acquisition précise de l'hémodynamique pour déterminer les variations d'efficacité de l'hémodynamique, ces moyens étant susceptibles de commander l'envoi et éventuellement les paramètres de l'impulsion ou de la salve et/ou l'application d'une dose de médicament perfusée externe pilotée
ou à partir d'un réservoir de médicament implanté jusqu'à obtention d'un degré de performance hémodynamique, par exemple programmé ou déterminé à l'avance, ou bien d'une façon proche de celle qui a produit l'hémodynamique la plus favorable pour le patient à un moment donné.

Différents aspects de l'invention vont maintenant être décrits.

### Contrôle de la STIO

D'une façon générale, et notamment dans les insuffisances sévères ou aiguës, les variations présentes des zones réfractaires électriques (ZRE), des zones réfractaires mécaniques (ZRM), des seuils d'excitabilité, de l'hémodynamique et des comportements métaboliques ne peuvent pas, à elles seules, prévoir l'évolution de l'état fonctionnel intramyocardique du couplage électromécanique (CEM). Celui-ci (CEM) est observable uniquement par les comparaisons temporelles spécifiques, précises et individualisées que peut donner le dispositif selon l'invention, notamment sous forme de simulateur analyseur (SIMA), sans lesquelles les rapides défaillances myocardiques peuvent rester imprévisibles et imparables par la STIO.

### Maintien optimal et continu des paramètres de la STIO par rapport aux variations des seuils d'excitabilité myocardiques

Dans une forme préférée de l'invention, on effectue une surveillance continue du seuil d'excitabilité du coeur à l'aide d'une diminution périodique de l'énergie stimulante utilisée conformément au brevet Zacouto FR-A1-1,237,702, PV 651.632 du 11 juillet 1953 et permettant un réajustement instantané des paramètres de stimulation. Une manière de réaliser ceci peut consister à diminuer progressivement ou brusquement l'impulsion stimulante du train des impulsions jusqu'à recapture de cette impulsion. Durant les périodes où cette impulsion reste inefficace, l'impulsion qui la suit dans son train prend la commande du coeur. Lorsqu'une diminution de l'énergie stimulante reste suffisamment fiable, la STIO peut être programmée pour enclencher une stimulation, par exemple, à amplitude diminuée, ce qui diminue aussi le risque de provoquer des extrasystoles. On peut également, par exemple, diminuer d'un tiers l'amplitude proche du seuil des trois ou quatre premières impulsions d'un train comportant six ou huit impulsions pour observer si cette stimulation ciblée infra-liminaire favorise ou non le seuil d'excitabilité myocardique. Si ce seuil résulte diminué, on pourra quantifier cette « facilitation », par exemple, en pourcentage d'amplitude diminuable et, par une répétition périodique de cet examen, mesurer sa durée de facilitation.

A l'inverse, un décrochage de l'impulsion stimulante ou du train entier de stimulation enclenchera une élévation instantanée de l'amplitude énergétique de l'impulsion devenue infra-liminaire ou de l'ensemble du train stimulant du prochain cycle cardiaque. Pour les cycles cardiaques suivants, on peut avantageusement utiliser une rampe d'impulsion à voltage augmentant progressivement afin de déterminer, par mesure automatique à l'intérieur de chaque cycle cardiaque, pour le programme du logiciel orthorythmique inotropique, le nouveau seuil d'excitabilité myocardique à partir duquel un nouveau processus de contrôle et d'intervention sur l'amplitude des impulsions stimulantes s'enclenchera.

Ainsi, la STIO réalise une adaptation instantanée permanente optimale des intensités stimulantes selon leurs intervalles de couplage par rapport à l'onde R précédent, qu'il s'agisse aussi bien d'une diminution que d'une augmentation spontanée des seuils d'excitabilité myocardiques.

Lorsque l'on cherche à préciser un seuil d'excitabilité myocardique, on s'aperçoit qu'il ne s'agit pas d'une limite ponctuelle mais d'une zone se trouvant entre l'amplitude infra-liminaire où l'on n'observe aucune stimulation propagée, et l'amplitude supra-liminaire où l'on observe un entraînement propagé constant. L'étendue de cette zone d'excitabilité inconstante est variable.

De façon particulièrement préférée, le dispositif comporte des moyens pour donner à une impulsion destinée à la stimulation électrique, une amplitude supérieure d'au moins 30 %, et de préférence entre 30 % et 90 % de l'amplitude de la dernière impulsion infraliminaire constatée.

Les paramètres sur lesquels le dispositif agit peuvent être simplement un rythme ventriculaire programmé et/ou un réglage automatique du début ou de la fin ou de la durée de la salve ou le nombre ou les caractéristiques (notamment largeur, polarité, intensité) des impulsions dans la salve, ou encore une localisation de l'envoi de la salve sur différentes électrodes de stimulation. Par exemple, on peut réduire progressivement une salve à une seule impulsion. Pour cela, on peut tâter périodiquement avec, au moins, une deuxième impulsion qui se déplace progressivement de, par exemple, 25 ms en avant de l'impulsion stimulante et recule ensuite pas à pas, par exemple de 4 m/s à chaque cycle, pour automatiquement mesurer le début de la zone non réfractaire. Quand l'impulsion exploratrice recule vers l'impulsion stimulante, on fait reculer cette impulsion elle-même jusqu'à obtention du début de baisse de la courbe pression/volume ventriculaire ou d'augmentation de la consommation d'oxygène ou de la sécrétion et accumulation membranaire d'électrons ou, par exemple, de catécholamines proximales ou d'acide lactique, cette position correspondant au dépassement de la fin de la zone réfractaire mécanique à contraction active maximale dite ZRMM.

De préférence, il est prévu des moyens de stimulation anti-tachycardique et des moyens sensibles aux extrasystoles pour stopper automatiquement ladite stimulation en cas de survenue d'une instabilité hémodynamique trop grande ou d'arythmie électrique répondant à des critères prédéterminés.

La stimulation électrique du coeur selon l'invention permet d'obtenir, à chaque cycle cardiaque (CC), une stimulation pairée (STP) ou couplée (STC) inotrope optimale (STIO) par ajustements très précis des impulsions par rapport aux zones fonctionnelles réfractaires électriques (ZRE) et myocardiques (ZRM) et aux réactions métaboliques correspondantes et dans l'espace par rapport aux localisations cardiaques des électrodes permettant d'obtenir la meilleure hémodynamique, ou efficacité antiarythmique.

Ces zones fonctionnelles peuvent varier significativement à chaque cycle, notamment en cas de souffrances du myocarde. Pour contrôler à chaque cycle les ZRE et ZRM et l'hémodynamique correspondante, on peut analyser en permanence et en temps réel ces zones grâce, par exemple, aux moyens décrits dans les brevets Zacouto US No 4, 052,991 et 3,857,399.

Le dispositif peut comprendre des moyens pour réduire progressivement une salve à une seule ou un petit nombre d'impulsions, notamment en tâtant périodiquement avec, au moins, une deuxième impulsion qui se déplace progressivement en avant de l'impulsion stimulante pour automatique mesurer le début de la zone non réfractaire, de sorte que, quand l'impulsion exploratrice recule vers l'impulsion stimulante, cette dernière peut être reculée, notamment périodiquement en cas d'instabilité du fonctionnement jusqu'à obtention du début de baisse de la courbe pression/volume ventriculaire ou d'augmentation de la consommation d'oxygène ou d'un paramètre correspondant, notamment la sécrétion membranaire d'électrons, pH local ou corps cétoniques, cette position correspondant au dépassement de la fin de la zone réfractaire mécanique à contraction maximale (ZRMM).

Il peut comprendre des moyens pour passer d'une stimulation pairée à une stimulation couplée, c'est-à-dire à un rythme entièrement stimulé lesdits moyens étant sensibles aux moyens d'acquisition de l'électrocardiogramme et/ou de l'hémodynamique, et/ou du métabolisme myocardique.

### Inhibition instantanée des extrasystoles ou arythmies pendant la STIO

Le dispositif selon l'invention peut également être agencé pour traiter les extrasystoles et les arythmies qui peuvent apparaître, soit spontanément, soit sous l'effet du fonctionnement du dispositif.

Une condition pour le bon fonctionnement de la STIO consiste à détecter instantanément les extrasystoles précoces qui peuvent suivre ou accompagner un train d'impulsion et de réagir également instantanément en provoquant une stimulation ou un train de stimulation dès le début de la détection d'une telle extrasystole. La détection instantanée d'une extrasystole précoce nécessite un enregistrement ECG, de préférence indépendant de sa propagation directionnelle intramyocardique qui est lente (1 m/sec) avant d'arriver à l'électrode détectrice intracardiaque. Pour cela, on peut utiliser une détection par électrodes juxta- ou extracardiaques ou intracavitaire ventriculaires, de préférence sans contact avec le myocarde, qui sont sensibles à la vitesse de propagation des variations du champ électrique du coeur qui est proche de la vitesse de la lumière.

Dès la détection confirmée d'une extrasystole prématurée, le programme anti-arythmique déclenchera instantanément une stimulation de préférence en salve à partir de toutes les électrodes stimulantes disponibles afin de provoquer aussitôt un complexe de fusion entre les propagations des dépolarisations stimulées et extrasystoliques. Ce complexe de fusion bloquera la propagation de la dépolarisation extrasystolique et permettra souvent la suppression des ectopies prématurées myocardiques. On peut aussi, lorsque des ectopies myocardiques précoces surtout répétées menacent l'efficacité de la STIO, raccourcir instantanément et temporairement les diastoles pour fermer les intervalles non réfractaires des cycles cardiaques durant lesquels l'amorçage des extrasystoles précoces peut intervenir.

Dans une telle forme de réalisation, permettant de traiter les arythmies et les extrasystoles isolées, le dispositif selon l'invention peut comprendre une pluralité d'électrodes disposées en différents emplacements du muscle cardiaque et/ou à distance du coeur et des moyens d'acquisition sensibles aux signaux électriques apparaissant sur les électrodes et à distance d'elles pour constater, à un stade précoce, la survenue d'une extrasystole électrique dans une zone myocardique proche de celles des électrodes initialement concernées. Les moyens permettant d'adresser les impulsions de stimulation sont alors rendus sensibles à une telle constatation pour émettre instantanément dans une électrode proche ou dans une pluralité d'électrodes, une impulsion ou une salve de stimulation dont la propagation électrique dans le myocarde se dirige vers la zone myocardique affectée par l'extrasystole, et entraîner un complexe de fusion entre elles qui peut bloquer la propagation de l'extrasystole.

Afin de pouvoir détecter suffisamment tôt, et surtout de pouvoir adresser immédiatement une impulsion de stimulation à proximité du territoire en train d'être concerné par la dépolarisation extrasystolique, le dispositif est, de préférence, relié à une pluralité d'électrodes, telles que des électrodes au niveau du sinus coronaire, des électrodes du septum et des parois libres ventriculaires, et de préférence juxtacardiaques, extracardiaques ou intracavitaires des électrodes, le dispositif étant agencé pour que les moyens d'acquisition détectent l'électrode près de laquelle l'extrasystole est originaire, les moyens d'envoi d'une impulsion de stimulation ou d'une salve étant agencés pour adresser la stimulation au moins à partir d'une électrode éloignée de l'origine de l'extrasystole.

Ainsi, lorsque la STIO est dérangée par des survenues intempestives d'extrasystoles apparemment provoquées par elles-mêmes, leur suppression est favorisée par une réponse électrique instantanée capable d'occuper au plus tôt les zones encore non-réfractaires du myocarde. A cette fin, il est préférable de détecter les extrasystoles non pas au niveau de cathéters bipolaires intra-cardiaques mais au niveau extra-cardiaque. En effet, la détection intra-cardiaque ne « voit » venir l'extrasystole qu'avec un retard correspondant à sa vitesse de propagation à partir de sa naissance jusqu'à l'arrivée sous l'électrode de détection qui se déroule avec une vitesse intra-myocardique de l'ordre d'1 m/seconde. Par contre, une électrode plus extérieure au coeur « voit » la dépolarisation myocardique se propager avec une vitesse proche de celle de la lumière. Il convient donc de détecter les extrasystoles, et notamment celles considérées par la programmation comme dangereuses, à l'aide d'électrodes juxta- ou extracardiaques ; dès l'apparition d'une telle détection confirmée comme critique, il convient de provoquer une salve de stimulations au niveau d'une ou de plusieurs ou de préférence de toutes les électrodes stimulantes. Si on dispose de plusieurs électrodes stimulantes, il convient de stimuler simultanément à partir d'elles de manière à provoquer des complexes QRS fusionnés qui bloquent les espaces myocardiques non réfractaires empêchant ainsi une propagation de nouvelles extrasystoles. Ce système doit agir de préférence à l'intérieur d'un seul cycle cardiaque mécanique.

L'enregistrement séquentiel des événements extrasystoliques, notamment les dangereuses, permet de créer des registres conformément au brevet Zacouto US N° 5,306,293 ; ceci ouvre la possibilité, en cas de répétition de crise, d'exercer une stimulation préventive, notamment une accélération temporaire du rythme de base avant la nouvelle survenue de ces extrasystoles.

Dans une autre forme de réalisation, dans laquelle le dispositif comprend des moyens pour détecter rapidement une extrasystole, les moyens du dispositif sont sensibles à l'acquisition de cette extrasystole pour diminuer ou même supprimer, de préférence temporairement, les phases diastoliques électriques (D). Ceci s'effectue soit en augmentant le rythme de stimulation dans un coeur sous contrôle électrique du dispositif, soit en prenant le contrôle pour adresser une impulsion de stimulation, au tout début de la diastole électrique.

Dans ces derniers cas, le dispositif selon l'invention passe de l'état de stimulation pairée à l'état de stimulation couplée.

On peut ainsi empêcher l'installation d'une tachycardie tout en maintenant à la fois un rythme très inférieur au rythme tachycardique premier, et en bénéficiant d'un effet de renforcement post extrasystolique.

Un tel dispositif est particulièrement efficace pour contrôler les arythmies naissantes provoquées par la stimulation ou d'origine proche de la ou des zones dans lesquelles sont adressées les impulsions ou salves de stimulation.

Dans une autre forme de réalisation, on peut, en cas d'apparition d'une arythmie, augmenter temporairement le nombre et/ou l'intensité des impulsions de la salve pour renforcer l'effet de stabilisation sur les membranes cellulaires myocardiques ou administrer automatiquement, par programme STIO, une drogue en perfusion ou à partir d'un réservoir de médicament implanté (voir notamment Zacouto, US-A-5,305,745).

Dans d'autres cas, le dispositif peut être agencé, en cas d'arythmie, pour, au contraire, diminuer l'intensité des impulsions de la salve, le dispositif surveillant alors si l'arythmie se poursuit. Dans ce cas, l'intensité de la stimulation pourra être augmentée.

Dans une autre forme de réalisation, le dispositif selon l'invention peut être utilisé de façon efficace en cas de fibrillation auriculaire, entraînant une arythmie ventriculaire. Le dispositif selon l'invention est agencé pour acquérir le mécanogramme cardiaque, de préférence jusqu'à l'occurrence d'un cycle assez long pour obtenir une bonne contraction myocardique par rapport au rythme instantané en cours, définie, par exemple, par un seuil pré-programmé et les moyens de stimulation envoient alors une impulsion ou une salve de stimulation à un instant situé à l'intérieur du plateau systolique de la courbe du mécanogramme, c'est-à-dire du sommet, relativement aplati de la courbe du mécanogramme.

Le dispositif ayant acquis un cycle immédiatement antérieur, on peut situer la période réfractaire électrique ZRE en pourcentage de la durée du cycle antérieur, l'impulsion étant lancée dans ledit plateau de la courbe hémodynamique de préférence un peu avant la fin de cette zone réfractaire mécanique maximale contractée, par exemple 30 à 40 ms avant la fin de cette zone ZRMM, laquelle fin de zone peut être détectée par un microcapteur intramyocardique de pression locale à l'endroit proche de l'électrode active pour l'envoi d'une salve d'impulsions espacées de 15 ms, après quoi le dispositif stimule immédiatement, de préférence de façon multipolaire, après la fin de la zone réfractaire électrique qui vient d'être prolongée, annulant ainsi la diastole électrique. De façon avantageuse, ceci peut être effectué sur des électrodes localisées à proximité de la zone ayant vu apparaître l'arythmie en utilisant des électrodes disposées comme décrit ci-dessus.

Par exemple, le dispositif peut stimuler d'abord l'oreillette, par une électrode intra-auriculaire, puis, si besoin, le ventricule par une électrode intraventriculaire, selon une succession bien connue dans les pacemakers de type DDD. On peut ainsi, par une stimulation STIO au niveau auriculaire, régénérer ou améliorer le tissu myocardique auriculaire.

Dans une autre forme de réalisation, lorsque le faisceau de His du patient est plus ou moins bloqué, par exemple par la Digoxine, on peut diminuer progressivement la fréquence de stimulation en surveillant les contractions prématurées d'origine auriculaire.

Le dispositif selon l'invention peut être intégré à un défibrillateur automatique (DAI), par exemple un défibrillateur implanté, et être mis en oeuvre, notamment après un choc de défibrillation, ou bien encore à titre préventif d'arythmies graves.

Les défibrillateurs implantés comportent actuellement des moyens antitachycardiques, du type anti-arythmique selon le brevet US 3,857,399 Zacouto, et le dispositif selon l'invention peut, par exemple, être actionné lorsque le dispositif antitachycardique ne parvient pas à réduire rapidement une tachycardie.

### Stimulation inotrope pendant la zone réfractaire musculaire de contraction maximale (ZRMM)

Dans un mode préféré d'optimisation, le logiciel pilotant le dispositif selon l'invention est programmé pour automatiquement trouver l'étroite zone efficace ZRMM dans chaque cycle concerné, et éventuellement, réduire ensuite les différents paramètres des salves d'impulsions, notamment leur nombre, jusqu'à aboutir, si possible, à une seule impulsion parfaitement ciblée. Etant donné qu'une telle stabilité du fonctionnement de la STIO est inexistante ou inconstante, surtout en cas de souffrance myocardique, la programmation qui détecte en permanence les paramètres mécaniques, de préférence avec leur consommation métabolique (oxygène ou équivalent), devra inverser, en cas d'instabilité suffisante ou croissante, le processus de réduction des salves afin de retrouver sans délai avec des salves l'emplacement de la nouvelle zone étroite utile du cycle. Une méthode rapide pour trouver la zone ZRMM consiste à détecter, à l'intérieur d'une salve, l'impulsion efficace qui produit une dépolarisation propagée et de réduire progressivement sa distance par rapport à l'impulsion qui la précède jusqu'à ce qu'elle décroche et n'entraîne plus l'électrogénèse visible sur l'électrocardiogramme, qui sera, à ce moment, provoquée par l'impulsion suivante de la salve. On connaît ainsi précisément le début de la zone utile et on peut ajouter environ 20 ms, et considérer que cet intervalle représente la zone utile à une période donnée. Il convient de répéter périodiquement cette recherche automatique, très anodine pour le patient, afin de ne pas perdre l'exacte localisation de la ZRMM au cours de ses variations. On peut aussi, après avoir trouvé l'impulsion efficace de la salve d'impulsion, réduire le voltage ou la largeur des autres impulsions de manière à les rendre légèrement infra-liminaires, ce qui peut maintenir leur effet anti-arythmique et de sentinelle fonctionnelle, car une impulsion infra-liminaire proche du seuil influence souvent ce seuil en le diminuant pour une courte durée (effet de facilitation). La mesure automatique de ce phénomène peut se faire en diminuant légèrement l'intensité de la ou des impulsions qui précèdent l'impulsion stimulante dans une salve et en diminuant progressivement l'intensité, le voltage ou la largeur de l'impulsion stimulante jusqu'à son décrochage fonctionnel, l'impulsion suivante de la salve gardant une intensité stimulante ; on peut ensuite diminuer aussi progressivement l'intensité de cette impulsion pour mesurer la variation de son seuil d'excitabilité et, en la décalant progressivement dans le temps, on peut ainsi mesurer la durée et l'intensité de cet effet de facilitation.

En variante, afin de détecter la zone de contraction maximale ZRMM, le dispositif peut comprendre des moyens de mesure précis du volume du coeur ou d'une partie du coeur par impédance électrique, ou encore par échographie localisée, par exemple en comprenant une sonde ultrasonore échographique de mesure tissulaire, orientée vers une paroi cardiaque pour mesurer son déplacement, permettant d'obtenir en temps réel les variations de volume myocardique, et ainsi déterminer le moment de la contraction maximale.

L'invention a ainsi pour objet un dispositif de stimulation et/ou renforcement du muscle cardiaque et/ou des cellules myocardiques permettant un accroissement significatif de la performance hémodynamique du coeur et/ou le traitement des tachycardies, tachyarythmies ou fibrillations auriculaires, comprenant :
- des moyens d'acquisition précise de l'hémodynamique cardiaque (5, 6) et comprenant un moyen de détection instantané de la zone réfractaire myocardique maximale ZRMM en un emplacement précis du myocarde,
- et des moyens pour adresser au moins une impulsion de stimulation et, de préférence, une salve d'impulsions de stimulation à partir de la région locale dans laquelle on détecte la survenue de la zone ZRMM.

Il comprend, de préférence, des moyens d'acquisition automatique (1, 2, 3) du rythme cardiaque et optionnellement de son origine, permettant notamment de connaître l'intervalle entre au moins les deux dernières ondes R (provoqué ou spontané) du cycle cardiaque qui vient de s'achever, et des moyens (4) permettant de déterminer continuellement en temps réel la durée de la période réfractaire électrique (ZRE) qui suit la dernière onde (R) d'un cycle cardiaque, ledit dispositif étant agencé pour détecter si un signal électrique de dépolarisation a bien été produit par ladite stimulation dans ladite zone ZRMM.

Ces moyens permettent de détecter et de mémoriser la durée de ladite zone réfractaire myocardique maximale ZRMM et d'adresser ladite impulsion de stimulation ou salve d'impulsions de stimulation locale après une courte durée après le début, dans le cycle en cours, du début de ladite zone ZRMM et avant la fin estimé de ladite zone par mémorisation de ladite durée dans les cycles précédents.

Conformément à l'invention, l'impulsion ou au moins une impulsion d'une salve de stimulation, tombe dans ladite zone (ZRMM), sensiblement à l'endroit du myocarde où l'on détecte la survenue de ladite zone (ZRMM).

Le dispositif peut comporter des moyens de mesure de pression intracavitaire implantés et susceptibles de détecter une zone de pression maximale dans le plateau du mécanogramme cardiaque et auxquels sont sensibles lesdits moyens d'envoi d'une impulsion ou d'une salve de stimulation.

De préférence, il comporte au moins un capteur de détection de pression intramyocardique. Ledit capteur de pression intra myocardique est situé dans le septum intra-auriculaire et/ou dans une paroi cardiaque libre ou intra-ventriculaire.

En variante, on peut prévoir des moyens de mesure de la variation de volume du coeur ou d'une partie du coeur, détectant et mémorisant l'intervalle du cycle où ledit volume a atteint et maintient sa valeur minimale, lesdits moyens pour adresser une impulsion ou salve de stimulation étant sensibles auxdits moyens de mesure.

On peut ainsi prévoir des moyens de détection de la consommation du coeur en oxygène et/ou un équivalent, notamment pH local ou concentration des corps cétoniques, et des moyens pour détecter la zone de contraction cardiaque maximale (ZRMM) par estimation en détectant, lors d'un ou plusieurs cycles préalables, la position relative, dans le cycle et/ou dans le mécanogramme, de l'impulsion ou de l'impulsion d'une salve ayant généré un renforcement post-extrasystolique (RPE) avec une consommation minimale d'oxygène par rapport au débit sanguin mesuré par minute.

### Analyse comparative avec simulation

Une STIO de sécurité en Clinique exige de disposer de méthodes de protection efficaces contre les extrasystoles interférentes et les variations spontanées des zones réfractaires du myocarde qui peuvent être augmentées par la stimulation en salves elle-même.

L'importance et la rapidité des variations des zones réfractaires électrique et mécanique du coeur peuvent s'expliquer par l'effort énergétique supplémentaire que la STIO demande à chaque contraction myocardique. L'organisme tend à éviter cet effort, imposé par un ciblage très précis des impulsions dans chaque cycle cardiaque, en modifiant à la fois les durées et seuils d'excitabilité de ces zones. Mais la simple adaptation, même parfaite, aux différents paramètres myocardiques ne suffit pas toujours pour connaître le débit sanguin de chaque contraction car les variations des paramètres intrinsèques (couplage électro-mécanique intracellulaire) de l'appareil contractile restent inconnues. Il s'ensuit qu'une analyse précise, constante et comparative par un « simulateur » du débit sanguin réel, en temps réel, par rapport aux cycles antérieurs avec monitorage et possibilité d'intervention automatique sur la STIO reste nécessaire.

Il faut savoir qu'en cas d'insuffisance cardiaque grave ou aiguë le maintien permanent d'une STIO devient plus difficile et il convient de pouvoir prévoir l'évolution, surtout à courte échéance, des paramètres cardiaques critiques. A cette fin, on procède à une mémorisation des variations des paramètres qui ont précédemment provoqué un risque de décrochage injustifié de la STIO et on stimule de façon à essayer d'éviter de pareilles concordances ; en cas de décrochage justifié, par exemple arythmies ou défaillance purement myocardique critique, la STIO sera programmée pour s'arrêter instantanément avec émission d'alarme et analyse des paramètres cardiaques responsables, et au besoin défibrillation ou stimulation anti-tachycardique ou injection automatique de drogues. Ces fonctions de sécurité peuvent être assurées par un « analyseur-simulateur » (SIMA) utilisant notamment l'analyse comparative entre, d'une part, les courbes ECG et hémodynamiques et/ou métaboliques simulées par rapport aux données du patient, et, d'autre part, réelles enregistrées en temps réel et superposées.

L'invention de la STIO, en s'adaptant sans délai à ces diverses variations d'échappements fonctionnels spontanées du coeur, assure une continuité de son effet inotrope optimal.

L'analyse par simulation théorique adaptée à chaque patient et sa différence par rapport au tracé réel des paramètres cardiaques et vasculaires en temps réel et différé permet de montrer continuellement la réactivité fonctionnelle du myocarde envers sa stimulation par le pacemaker orthorythmique inotrope. Cette stimulation STIO permet de visualiser instantanément et d'agrandir à volonté les différences entre les tracés souhaités et les tracés réels ainsi que leurs variations ce qui permet, à son tour de réajuster, si besoin, instantanément et automatiquement des paramètres de la stimulation électrique. Il est ainsi possible de hachurer, de colorier différemment et de quantifier continuellement de façon automatique les différences de soustraction de ces tracés. Un réajustement manuel ne peut être aussi rapide et nécessite assez souvent la compréhension préalable du mécanisme physiopathologique en cause.

Compte tenu des informations que l'on possède sur le coeur d'un patient et de son fonctionnement, on peut ainsi établir une simulation des effets que l'on est théoriquement en droit d'attendre par mise en oeuvre du procédé selon l'invention et comparer cette simulation et la stimulation réellement effectuée.

Dans cette forme de réalisation, le dispositif comporte :
- des moyens pour acquérir des informations relatives à l'électrocardiogramme d'un patient, y compris le rythme cardiaque,
- des moyens pour acquérir des informations relatives à la performance hémodynamique du patient ;
- des moyens d'analyse sensibles auxdits moyens d'acquisition pour simuler l'effet d'une stimulation pairée ou couplée inotrope, adaptée au coeur du patient.

Il présente avantageusement, en outre, des moyens permettant de comparer automatiquement lesdites informations acquises relatives à la performance hémodynamique du patient à l'effet simulé sur ladite performance d'une stimulation pairée inotrope.

De préférence, lesquels lesdits moyens d'acquisition et de comparaison permettent l'acquisition et la comparaison des informations cycle par cycle.

Parmi les paramètres permettant de déterminer l'état du coeur chez un patient et sa réactivité, on retiendra son électrocardiogramme à l'état spontané ou bien sous assistance d'un pacemaker, par exemple un pacemaker orthorythmique. On retiendra également des informations sur la performance hémodynamique du muscle et sa réactivité aux stimulations électriques. Les différents paramètres et moyens permettant de les acquérir, et notamment pour l'efficacité cardiaque, y compris débit, volume et pression, consommation d'oxygène et autres, ont été définis précédemment et sont aussi décrits dans les documents incorporés par référence.

De préférence, lesdits paramètres comprennent au moins l'un des paramètres suivants relatifs à la contraction :
- pente (dp/dt) des phases de montée et/ou descente de la pression intracavitaire et/ou intramyocardique ;
- durée du « plateau » de pression systolique, correspondant à l'éjection systolique ;
- durée de la systole (contraction cardiaque) ;
- durée de la diastole (phase de remplissage, y compris phases initiale motrice, rapide et lente) ;
- rapport entre les durées de diastole et de systole ;
- qualité de la diastole, notamment dépression de remplissage ;
- couplage électromécanique (durée séparant le début d'un complexe QRS du début de la systole mécanique qu'il provoque).

De préférence, le dispositif comporte des moyens permettant une comparaison des informations relatives à l'électrocardiogramme et à la performance hémodynamique d'une simulation de stimulation pairée ou couplée inotrope, avec des informations correspondantes acquises lors d'une stimulation inotrope réelle ultérieure identique ou similaire.

De préférence, il comporte des moyens pour acquérir ou calculer un niveau de seuil de valeurs des informations de la performance hémodynamique simulée adaptée au patient, et en cas de dépassement du seuil, provoquer une stimulation réelle, pairée ou couplée inotrope identique ou similaire.

Lesdits moyens pour acquérir les informations relatives à l'électro-cardiogramme du patient et à la performance hémodynamique du patient, sont agencés pour acquérir ces informations à différents rythmes.

De préférence, le dispositif est agencé pour imposer temporairement au coeur du patient des rythmes variant par paliers ou progressivement, pendant lesquels on acquiert lesdites informations relatives à l'électrocardiogramme et lesdites informations relatives à la performance hémodynamique correspondante.

De préférence, lesdits moyens sensibles auxdits moyens d'acquisition sont agencés pour simuler les effets de plusieurs stimulations pairées ou couplées inotropes à différents rythmes cardiaques.

On préfère effectuer ladite comparaison pendant un nombre limité de cycles de la stimulation inotrope réelle et, en cas de constatation de l'absence d'un niveau satisfaisant de performance hémodynamique, mettre fin à la stimulation en cours.

Ledit nombre de cycles peut être, par exemple, de l'ordre d'une dizaine ou inférieur, notamment 1, 2 ou 3 cycles.

Le dispositif est, de préférence, agencé pour, en cas de constatation d'un accroissement de performance hémodynamique initial suffisant, poursuivre la stimulation inotrope et détecter et mémoriser si l'augmentation de performance hémodynamique initial se maintient et/ou augmente encore progressivement pendant un nombre plus grand de cycles.

Ledit nombre plus grand de cycles est, de préférence, d'au moins une centaine de cycles.

Lesdits moyens d'analyse peuvent être agencés pour inclure les effets inotropes médicamenteux susceptibles d'interférer avec l'effet de la stimulation inotrope électrique.

Le dispositif peut comprendre des moyens pour souligner visuellement et/ou quantitativement par calcul, les différences entre les courbes de la simulation et de la stimulation réelle correspondante.

De préférence, tous ces renseignements relatifs à l'électro-cardiogramme, au rythme cardiaque et aux caractéristiques hémodynamiques ou physiologiques, sont obtenus et mémorisés dans le dispositif selon l'invention, quel que soit le rythme cardiaque spontané ou stimulé du patient. Dans un tel cas, la simulation est affinée par l'analyse qui est faite en comparant les variations des paramètres, et notamment des paramètres énumérés ci-dessus, aux différents rythmes.

Ces modifications de rythme ou de réactivité cardiaques peuvent aussi être acquises en soumettant le patient à l'action de médicaments appropriés agissant par exemple sur le rythme et/ou la contraction cardiaque, par exemple la dopamine.

Ainsi, par exemple, le couplage électromécanique et la façon dont il peut varier lorsque le rythme varie, donnera une bonne indication sur la réactivité mécanique du coeur après stimulation. La pente de la montée en pression (dp/dt) donnera une indication de la capacité du muscle cardiaque à engendrer une élévation de pression alors que la pente de la descente de la pression pourrait être indicative de la compliance ou de l'élasticité du muscle cardiaque, et donc de sa capacité de remplissage lors de la diastole mécanique, selon le rythme.

Le dispositif selon l'invention est programmé pour utiliser les informations ainsi acquises et proposer, au moins pour le rythme spontané ou le rythme de base du patient, et de préférence pour d'autres rythmes, un électrocardiogramme et un mécanogramme simulant un résultat d'un fonctionnement sous une stimulation pairée ou couplée selon l'invention.

Un mécanogramme simulé permettra ainsi d'obtenir, notamment, le débit cardiaque simulé, et on peut alors comparer ce débit simulé au débit mesuré du patient en rythme spontané ou en rythme simulé de base s'il y a lieu, et estimer si la mise en oeuvre de la stimulation inotrope selon l'invention permettrait d'obtenir une augmentation significative, de préférence d'au moins 20 %, du débit conduisant à une amélioration significative du patient en insuffisance cardiaque. La réalisation de simulations à des rythmes différents permet d'estimer des débits cardiaques correspondant respectivement à ces rythmes et, le cas échéant, de sélectionner un rythme particulier, par exemple accéléré, pour un patient insuffisant cardiaque ayant un rythme spontané relativement bas, ou diminué, par exemple pour un patient ayant un rythme spontané élevé, que l'on peut espérer diminuer par la stimulation inotrope selon l'invention.

Les étapes suivantes du procédé selon l'invention consistent alors à appliquer au patient la stimulation inotrope choisie grâce à l'étape de simulation, à acquérir instantanément les résultats de cette stimulation, et à comparer ces résultats aux résultats théoriques de la simulation.

Le dispositif est, de préférence, programmé pour vérifier immédiatement les modifications de la performance hémodynamique au bout d'un nombre très réduit de cycles cardiaques, par exemple pendant 1, 2 ou 3 cycles mécaniques, ou pendant une ou quelques dizaines de cycles. Si aucune amélioration significative n'apparaît et ne se maintient, par exemple au bout de trois cycles, le dispositif stoppe la stimulation inotrope, ou, selon la programmation réalisée, essaye une stimulation inotrope dans des conditions différentes, par exemple à un rythme différent.

Dans le cas où l'on constate, au bout de ce faible nombre de cycles, une première amélioration réelle de la performance hémodynamique, la stimulation inotrope selon l'invention est poursuivie pendant au moins une centaine ou quelques centaines de cycles et le dispositif vérifie si l'accroissement de performance hémodynamique initiale se produisant tout au début de la stimulation inotrope, non seulement se poursuit mais augmente encore progressivement pendant ce plus grand nombre de cycles et sera mémorisé.

La comparaison entre l'augmentation simulée de performance hémodynamique et l'augmentation ou la modification réelle de la performance hémodynamique peut être obtenue, par exemple, en superposant les courbes hémodynamiques simulées et réelles et en mesurant les différences, à savoir au moins la différence de débit, et de préférence des différences sur d'autres paramètres, notamment certains ou tous les paramètres énumérés ci-dessus.

On pourrait décider, par exemple, que si la différence, notamment en matière de débit, est négative et se place au dessous d'un seuil, par exemple de 10 ou 20 % au dessous de la valeur du débit simulé, la stimulation inotrope qui a été mise en oeuvre n'est pas suffisante, le dispositif arrêtant alors la stimulation isotrope ou essayant une autre stimulation inotrope.

Si, au contraire, l'accroissement de la performance hémodynamique dépasse celle simulée, d'une valeur par exemple d'au moins 10 ou 20 %, on pourra envisager de contrôler la stimulation inotrope, par exemple en n'envoyant les impulsions ou salves de stimulation pairées ou couplées que pour certains cycles cardiaques et non à chaque cycle ou l'on pourra également utiliser les index de consommation d'oxygène ou d'autres index tels que définis dans les documents antérieurs incorporés par référence, afin de vérifier que l'accroissement de la performance hémodynamique ne se fait pas au détriment de la disponibilité de l'oxygène pour le patient.

Par ailleurs, selon le perfectionnement de l'invention, les résultats électriques et mécaniques de la stimulation inotrope selon l'invention seront enregistrés et traités avec ceux préalablement acquis pour effectuer la simulation, afin de préciser davantage les caractéristiques particulières du coeur du patient telles que définies, par exemple, par les différents paramètres précités, ce qui permettra ensuite d'obtenir des électrocardiogrammes et/ou courbes électromécaniques de simulation affinées.

Surtout lorsque la STIO est appliquée sur des patients souffrant d'une insuffisance cardiaque grave ou aiguë, on observe que la STIO peut s'avérer impossible à débuter ou qu'elle se décroche au cours de son fonctionnement. Afin de prévoir et de s'opposer à ces graves incidents, il convient d'analyser avec précision la cause de ces non fonctionnements de la STIO. Il s'agit souvent d'une non superposition adéquate entre les zones réfractaires électrique (ZRE) et mécanique (ZRMM) qui peuvent se rétrécir ou se déplacer l'une par rapport à l'autre de telle façon que la durée de la zone critique efficace (ZCE) est pratiquement annulée ou plus fréquemment réduite, par exemple, à 10 ms. Dans ce cas, si la salve de stimulation de la STIO est constituée d'impulsions dont l'intervalle est de 15 ms, il devient impossible d'assurer une stimulation inotrope continue et le patient se trouve exposé à un épuisement de ses réserves d'oxygène.

Selon une variante de l'invention, et afin d'éviter cette défaillance grave de la STIO, il convient de mesurer avec précision sensiblement au même endroit du coeur, et au besoin en permanence, les durées et superpositions temporelles de ces ZRE et ZRMM ; cette fonction peut être assurée par le simulateur-analyseur (SIMA). Lorsque le SIMA enregistre un rétrécissement critique de la ZCE, il pourra automatiquement, sans délai, rapprocher convenablement les impulsions des salves de façon à essayer de faire cibler régulièrement au moins une impulsion à l'intérieur de la ZCE. Dans le cas d'une impossibilité d'atteindre régulièrement la ZCE, le SIMA stoppe, par programme, sans délai, la STIO. Un rapprochement permanent des impulsions entre elles au-deçà de 10 ms empêche, dans l'état actuel de l'électronique, les mesures de la ZRE et comporte d'autres inconvénients électroniques et possiblement physiologiques.

De préférence, le dispositif comporte :
- des moyens pour mesurer en permanence, dans un emplacement du coeur, la zone réfractaire électrique ZRE,
- des moyens disposés sensiblement dans la région du coeur pour mesurer avec précision les zones de contraction myocardique maximale ZRMM,
- le dispositif étant agencé pour acquérir, à partir desdits moyens, la superposition temporelle, dans un même cycle, desdites zones ZRE et ZRMM et déterminer la zone non commune et postérieure à la zone ZRE, dite zone critique ZCE.

Les moyens de stimulation électrique sont agencés pour adresser immédiatement au moins une impulsion de stimulation dans ladite région du coeur, pendant ladite zone ZCE.

Selon un perfectionnement, lesdits moyens vérifiant la superposition temporelle sont sensibles à un décalage entre les zones ZRE et ZRMM pour adresser une impulsion de stimulation ou une salve fournissant au moins une impulsion à l'intérieur de la zone ZCE.

De préférence, lesdits moyens sensibles permettent de faire diminuer l'intervalle de temps entre deux impulsions d'une salve pour augmenter la probabilité d'avoir une impulsion pendant ladite zone ZCE.

De préférence, la durée entre deux impulsions d'une salve ne peut pas être réduite à moins d'une valeur de l'ordre de 10 ms.

De préférence, en cas d'impossibilité de faire parvenir au moins une impulsion à l'intérieur de la zone ZCE, le dispositif stoppe la stimulation couplée ou pairée.

Selon un perfectionnement, le dispositif mémorise les survenues et les durées temporelles des zones ZCE pendant une pluralité de cycles et on analyse leur évolution pour prévoir une éventuelle tendance à la suppression de ladite zone ZCE et, dans ce cas, mettre en oeuvre un traitement, notamment par perfusion de drogues ou modification du rythme de la STIO afin d'agir sur la durée des zones ZRE ou ZRMM ou leur chevauchement.

Les moyens mis en oeuvre par le dispositif selon l'invention sont, sauf s'ils sont autrement décrits, des moyens qui, dans leur individualité, sont classiques dans l'art, et décrits, par exemple, dans les différents documents incorporés par référence.

Ainsi, les moyens d'acquisition associent des électrodes, capteurs physiques ou biochimiques connus à des moyens de mise en forme de signaux et des moyens de traitement informatiques, y compris microprocesseurs et mémoires, avec des logiciels que l'homme de l'art connaît ou peut produire de façon routinière, à partir du moment où il lit le fonctionnement décrit de l'invention. Il en est de même des moyens mis en oeuvre pour la stimulation, y compris leur source d'énergie électrique, et des moyens de traitement informatique sensibles, par exemple, à des rythmes, des durées, des seuils de détection et des courbes électriques ou hémodynamiques ou biochimiques acquises ou produites.

L'invention a également pour objet des procédés de traitement ou de prévention d'affections cardiaques comprenant les successions des étapes décrites ci-dessus, ces procédés mettant en oeuvre un dispositif tel que décrit dans la présente invention.

Ainsi, l'invention, dans un premier mode de mise en oeuvre, a pour objet un procédé de stimulation électrique du coeur, notamment pour traiter
ou prévenir les insuffisances cardiaques, et notamment l'insuffisance ventriculaire gauche, ou une tachycardie, une arythmie ou encore pour accroître significativement une performance hémodynamique du coeur, ledit procédé comprenant les étapes suivantes :
- implanter un dispositif de stimulation tel que décrit selon la présente invention ;
- acquérir automatiquement des informations sur le rythme cardiaque, de préférence, par acquisition de l'électrocardiogramme, de préférence à déroulement accéléré ;
- déterminer, de préférence à chaque cycle, de préférence en temps réel, la durée de la période réfractaire électrique (ZRE) qui suit la dernière onde R du cycle en cours ou d'un autre cycle antérieur proche ;
- et, au cycle suivant, adresser sensiblement sans délai avant la fin de ladite période réfractaire (ZRE) déterminée au moins une impulsion de stimulation, et de préférence une salve d'impulsion de stimulation, pour provoquer un allongement important de la période réfractaire (RZ) sans provoquer aucune sollicitation mécanique.

Dans un second mode de mise en oeuvre, un procédé selon l'invention, pour la stimulation du muscle cardiaque, notamment pour l'une des affections précitées, comporte les étapes suivantes :
- acquérir automatiquement des informations sur le rythme cardiaque, de préférence, par acquisition de l'électrocardiogramme ;
- déterminer, de préférence, à chaque cycle, de préférence à temps réel, la durée de la période réfractaire électrique (ZRE), qui suit la dernière onde R du cycle en cours ou d'un autre cycle antérieurement proche ;
- adresser, au cycle suivant, sensiblement sans délai à la fin de ladite période réfractaire (ZRE) déterminée au moins une impulsion, de préférence, une salve de stimulation ;
- acquérir automatiquement des informations sur l'hémodynamique cardiaque, de préférence la courbe pression/volume de la contraction cardiaque ;
- déterminer le renforcement de ladite hémodynamique ;
- en cas de renforcement nul ou trop faible, modifier le moment de l'envoi et/ou des paramètres de ladite impulsion ou salve jusqu'à l'obtention d'un degré de performance hémodynamique plus élevé.

Dans un mode de mise en oeuvre particulièrement préféré, on utilise des moyens (4) de détermination de la durée de la période réfractaire électrique (ZRE) sensibles à la détection de celle des impulsions d'une salve qui a déclenché un complexe R' et des moyens de détermination de la zone réfractaire mécanique maximale ZRMM, et l'on détermine, dans ladite zone ZRMM, une zone ZCE postérieure à la zone réfractaire électrique ZCE, et si ladite zone ZCE est existante, on adresse au moins une impulsion de stimulation dans ladite zone ZCE.

Dans un mode de mise en oeuvre particulier desdits procédés de l'invention, on laisse le coeur générer la systole électrique spontanée R qui provoque des contractions du muscle cardiaque. L'envoi de l'impulsion ou de la salve ou train d'impulsions peu avant la fin de ladite période réfractaire peut alors être dite « pairé » à ladite systole électrique spontanée R.

Dans une autre forme de réalisation, qui peut alors être utilisée et éventuellement en complément avec la précédente et notamment en cas de bradycardie, ou encore pour la réduction orthorythmique d'une tachycardie, on adresse régulièrement au coeur une impulsion de stimulation destinée à provoquer une électrosystole stimulée R entraînant la contraction myocardique et l'on envoie l'impulsion de couplage ou la salve de stimulation selon l'invention sensiblement sans délai à la fin de la période réfractaire, le coeur étant alors stimulé à un rythme électrique, ladite impulsion ou salve de stimulation étant alors dite « couplée » à l'électrosystole.

Dans les procédés selon l'invention, la détermination de la durée de la période réfractaire électrique (ZRE) peut être obtenue par les étapes telles que décrites à propos du fonctionnement du dispositif selon l'invention, par exemple par calcul et/ou par balayage, et/ou mesure.

Dans un mode de mise en oeuvre particulier des procédés selon l'invention dans lesquels on envoie une salve d'impulsions, la salve d'impulsions est adressée un peu avant la fin estimée de la période réfractaire (ZRE), la durée de la salve et le nombre d'impulsions étant tels qu'une stimulation électrique interviendra très vite après la fin de ladite période réfractaire (ZRE).

Dans un mode de mise en oeuvre perfectionné, on détecte celle des impulsions de la salve qui a déclenché une onde (R') qui prolonge la durée réfractaire (ZRE) et, s'il y a lieu, on modifie au cycle suivant, au moins une caractéristique de la salve.

Parmi ces caractéristiques, figurent notamment le moment du début de la salve, sa durée, le nombre d'impulsions, l'intervalle entre impulsions, et l'intensité des impulsions de la salve.

De préférence, on utilise aussi des moyens de stimulation anti-tachycardique et des moyens sensibles aux extrasystoles pour stopper automatiquement la stimulation programmée en cas de survenue d'une instabilité hémodynamique trop grande ou d'arythmie électrique grave répondant à des critères présélectionnés.

On peut utiliser, outre les moyens d'acquisition de rythme, les moyens de détermination de durée de zone réfractaire et les moyens d'envoi d'une impulsion ou d'une salve, des moyens (7,10) sensibles à l'acquisition précise de l'hémodynamique pour déterminer les variations d'efficacité de l'hémodynamique, ces moyens étant susceptibles de commander l'envoi et éventuellement les paramètres de l'impulsion ou de la salve, de préférence, d'une façon proche de celle qui a produit l'hémodynamique, la plus favorable pour le patient à un moment donné.

Lesdits moyens agissent sur des paramètres tels que : un rythme ventriculaire programmé et/ou un réglage automatique du début ou de la fin ou de la durée de la salve ou le nombre ou les caractéristiques, notamment largeur, intensité, polarité, densité, intervalle des impulsions dans la salve, ou encore une localisation de l'envoi de la salve sur une ou entre différentes électrodes de stimulation.

De préférence, on acquiert des paramètres métaboliques, notamment de consommation d'oxygène et/ou de ses équivalents, tels que la mesure des densités d'électrons de membrane cellulaire myocardique, ou d'augmentation de corps cétoniques, acide lactique, etc.

Dans une mise en oeuvre perfectionnée du procédé, on adresse une impulsion ou une salve de stimulation à l'intérieur de la zone (ZRMM) située à l'intérieur du plateau de la courbe de contraction du mécanogramme et pendant laquelle la contraction du myocarde est sensiblement maximale.

Ainsi, l'impulsion ou au moins une impulsion d'une salve de stimulation, tombe dans ladite zone ZRMM. De préférence, on acquiert ladite zone (ZRMM), automatiquement.

Dans un mode de mise en oeuvre, on mesure la pression intracavitaire par un capteur et on détecte et on choisit une zone de pression maximale dans le plateau systolique du mécanogramme cardiaque dans laquelle on provoque l'envoi d'une impulsion ou d'une salve de stimulation.

Mais, de préférence, on mesure la pression intramyocardique locale près d'une électrode de détection et de stimulation.

De préférence, on détecte la pression myocardique dans le septum intra-auriculaire et/ou intraventriculaire.

Dans un autre mode de mise en oeuvre, on mesure la variation de volume du coeur, ou d'une partie du coeur en détectant la zone où ledit volume a atteint et maintient sa valeur minimale.

L'invention a aussi pour objet un procédé, dans lequel :
- on acquiert des informations relatives à l'électrocardiogramme d'un patient, y compris le rythme cardiaque,
- on acquiert des informations relatives à la performance hémodynamique du patient ;
- et on utilise ces informations pour simuler l'effet d'une stimulation pairée ou couplée inotrope prolongée.

On compare lesdites informations acquises relatives à la performance hémodynamique du patient à l'effet simulé sur ladite performance d'une stimulation pairée inotrope.

De préférence, on procède à l'acquisition et la comparaison des informations cycle par cycle.

De préférence, on mesure au moins un des paramètres suivants relatif à la contraction cardiaque :
- pente (dp/dt) des phases de montée et/ou descente de la pression intracavitaire ou intramyocardique ;
- durée du « plateau » de pression systolique, correspondant à l'éjection systolique ;
- durée de la systole (contraction cardiaque) ;
- durée de la diastole (phase de remplissage) ;
- rapport entre les durées de diastole et de systole ;
- qualité de la diastole, notamment dépression de remplissage ou vitesse et amplitude des phases rapide et lente ;
- couplage électromécanique (durée séparant le début d'un complexe QRS du début de la systole mécanique qu'il provoque),
- décalage entre la courbe de contraction intramyocardique locale et la courbe de contraction intracavitaire ventriculaire globale.

On compare alors des informations relatives à l'électrocardiogramme et à la performance hémodynamique d'une simulation de stimulation pairée
ou couplée inotrope, avec des informations correspondantes acquises lors d'une stimulation inotrope réelle ultérieure identique ou similaire.

De préférence, on acquiert ou calcule un niveau de seuil de valeurs des informations de la performance hémodynamique simulée du patient, et en cas de dépassement du seuil, on déclenche une stimulation réelle, pairée ou couplée inotrope identique ou similaire.

De préférence, on acquiert les informations relatives à l'électrocardiogramme du patient et, à la performance et hémodynamique du patient, à des rythmes différents, notamment croissant ou décroissant par paliers ou progressivement.

De préférence, on utilise des moyens sensibles auxdits moyens d'acquisition agencés pour simuler les effets de plusieurs stimulations pairées ou couplées inotropes à différents rythmes cardiaques.

De préférence, on effectue ladite comparaison pendant un nombre limité de cycles de la stimulation inotrope réelle et, en cas de constatation de l'absence d'un niveau satisfaisant de performance hémodynamique, on met fin à la stimulation inotrope en cours.

En cas de constatation d'un accroissement de performance hémodynamique initial suffisant, on poursuit, de préférence, la stimulation inotrope et on détecte si l'augmentation de performance hémodynamique initiale se maintient et/ou augmente encore progressivement pendant un nombre plus grand de cycles.

On peut utiliser, dans l'analyse, les effets de médicaments à visée cardiovasculaire préalablement administrés ou en cours d'administration.

De préférence, on souligne visuellement et/ou quantitativement, notamment par superposition des courbes, les différences entre les courbes de simulation et de stimulation.

De préférence, on acquiert le mécanogramme cardiaque, notamment jusqu'à l'apparition d'un cycle assez long pour obtenir une bonne contraction myocardique, et on envoie alors une impulsion ou une salve de stimulation à un instant situé à l'intérieur du plateau de la courbe du mécanogramme (ZRMM), notamment un peu avant la fin de la zone réfractaire électrique (ZRE) après quoi on stimule immédiatement après la fin de la zone réfractaire électrique qui va provoquer une nouvelle zone réfractaire électrique.

On peut avantageusement définir un premier seuil d'augmentation de la performance hémodynamique globale/mn et/ou par contraction cardiaque, notamment le débit cardiaque, ledit seuil étant égal à au moins 15% ou de préférence de 25 % de la performance avant traitement, et on ajuste les paramètres de la stimulation jusqu'à l'obtention d'au moins ladite valeur de seuil.

Si l'on n'obtient pas l'augmentation selon ladite valeur de premier seuil au cours d'une période de l'ordre de 1 à 10 contractions, on préfère arrêter le traitement.

L'invention a également pour objet un procédé de traitement d'une insuffisance cardiaque aiguë ou sévère dans lequel :
- on acquiert automatiquement le rythme cardiaque, et notamment l'intervalle entre au moins les deux dernières ondes R (provoquées ou spontanées) d'un cycle cardiaque qui vient de s'achever,
- on détermine, de préférence, continuellement la durée de la période réfractaire électrique (ZRE) qui suit la dernière onde R dudit cycle,
- on adresse sensiblement sans délai, à la fin de la période réfractaire (ZRE), au moins une impulsion de stimulation et, de préférence, une salve d'impulsions de stimulation, la durée de la salve étant alors telle que, compte tenu de l'intervalle de répétition des impulsions dans la salve, une impulsion de stimulation de la salve se trouve adressée au coeur sensiblement sans délai après la fin de la période réfractaire, et
- on procède à la répétition de ces étapes pendant une série d'au moins trois contractions si l'on constate une première amélioration de la performance cardiaque,
- et, en l'absence d'une amélioration, on arrête automatiquement le procédé.

On compare avantageusement la performance mécanique du coeur totale notamment son débit sanguin et/ou la variation de volume ventriculaire, d'une part, avant la mise en oeuvre des étapes du procédé et, d'autre part, après la mise en oeuvre des étapes du procédé, et si l'augmentation de la performance du coeur est supérieure à 15 %, on met à nouveau en oeuvre les étapes de procédé ;

L'invention a également pour objet un procédé de réanimation cardiaque chez un patient souffrant d'une défaillance cardiaque sévère ou critique dans lequel :
- on acquiert automatiquement le rythme cardiaque, et notamment l'intervalle entre au moins les deux dernières ondes R (provoquées ou spontanées) d'un cycle cardiaque qui vient de s'achever,
- on détermine, de préférence, continuellement la durée de la période réfractaire électrique (ZRE) qui suit la dernière onde R dudit cycle,
- on adresse sensiblement sans délai, à la fin de la période réfractaire (ZRE), au moins une impulsion de stimulation et, de préférence, une salve d'impulsions de stimulation, la durée de la salve étant alors telle que, compte tenu de l'intervalle de répétition des impulsions dans la salve, une impulsion de stimulation de la salve se trouve adressée au coeur sensiblement sans délai après la fin de la période réfractaire, et
- on procède à la répétition de ces étapes au moins jusqu'à l'obtention d'une amélioration au moins progressive de la performance cardiaque détectable.

Les procédés de traitement ou de réanimation peuvent avantageusement aussi utiliser les autres étapes des procédés décrites précédemment.

L'invention a encore pour objet des procédés de traitement ou de prévention d'arythmies cardiaques notamment spontanées, mettant en oeuvre les étapes énumérées ci-dessus pour l'application du dispositif s'opposant aux arythmies. Elle a aussi pour objet des procédés de traitement ou de prévention d'arythmies cardiaques provoquées par, ou associées à la mise en oeuvre d'un dispositif selon l'invention, ledit procédé mettant en oeuvre les étapes correspondantes énumérées ci-dessus.

### Régénération du myocarde

L'invention a également pour objet un procédé de régénération physiologique et/ou anatomique et notamment cellulaire du myocarde dans le cas d'un muscle cardiaque insuffisant, ce procédé comprenant, de préférence, les étapes décrites ci-dessus.

Un tel procédé selon l'invention comprend les étapes suivantes :
- implanter dans le coeur, par exemple dans une oreillette droite ou gauche et/ou un ventricule droit ou gauche, des cellules de régénération, en position sous-endocardique ou intramyocardique, de préférence en plusieurs groupes d'amas cellulaires ou en nappe cellulaire,
- et assurer une stimulation selon l'invention, de préférence une stimulation pairée.

Un tel autre procédé selon l'invention comprend les étapes suivantes :
- obtenir et cultiver, *in vitro*, des cellules de régénération, de préférence sous forme de petits amas,
- mettre ces cellules en contacts de conduction électrique entre elles et avec un dispositif de stimulation électrique,
- adresser périodiquement des impulsions électriques auxdites cellules,
- détecter les réponses des dépolarisations et repolarisations électriques ou potentiels de membrane des cellules cultivées, lesdites cellules étant destinées à être implantées dans le coeur, de préférence en plusieurs groupes ou nappes de cellules, en position sous-endocardique ou intramyocardiques, une fois que leur détection électrique par micro-électrodes intracellulaires aura montré la capacité des cellules à une activité électromécanique rythmique adaptée au myocarde du receveur.

La stimulation peut être une stimulation électrique simple à un rythme, de préférence, de l'ordre d'un rythme cardiaque normal. Elle peut, de préférence, après une période initiale, être envoyée sous forme pairée ou couplée, une fois que les amas ou groupes de cellules en culture sont synchronisés et manifestent une période réfractaire électrique acceptable par rapport au myocarde du receveur.

Les cellules cultivées peuvent être de tout type, par exemple musculaires, myocardiques, embryonnaires ou cellules souches ou totipotentes ou multipotentes.

Ces cellules peuvent aussi avoir été obtenues par un procédé dans lequel on transfère, dans un ovocyte fonctionnel ou une cellule souche totipotente ou multipotente ou embryonnaire, un noyau ou une partie de noyau ou de chromosome étrangers ou de gènes, et l'on retire ce matériau nucléaire à un stade non achevé de la mitose pour le transférer dans une cellule, de préférence d'origine ou autologue, totalement ou relativement différenciée.

La régénération peut également porter sur des parties vasculaires, par exemple coronaires, mettant en oeuvre de tels moyens, avec une stimulation adaptée ou fonctionnement coronarien.

Afin d'accélérer et prolonger leur multiplication *in vitro* ou après transplantation, ces cellules, ou certaines d'entre elles, par exemple des cellules précurseurs myocardiques ou musculaires, ou autres, peuvent être modifiées génétiquement pour surexprimer la télomérase ou protéine Sir2, notamment par transfection par un vecteur viral ou rétroviral, ou autre vecteur, le gène de la télomérase ou protéine Sir2 transcriptase inverse (hTERT), en utilisant par exemple la technique décrite par Steven Goldman, Nature Biotech., February 16, 2004.

La STIO selon l'invention est aussi applicable à la régénération, au moins partielle, du muscle cardiaque, notamment chez l'insuffisant cardiaque chronique ou aigu par mise en oeuvre de la STIO, même en l'absence d'apport de cellules, de façon continue ou par périodes, pendant une durée longue, par exemple de quelques semaines à plusieurs mois ou plus.

L'invention a aussi pour objet un procédé de préparation de cellules vivantes, notamment végétales, animales et humaines, susceptibles d'être réimplantées à titre prophylactique ou thérapeutique, dans lequel on réalise le transport dans un ovocyte, de préférence non fécondé ou récemment fécondé, de mammifère homologue ou hétérologue, préalablement de préférence complètement ou partiellement vidé de son noyau, d'un noyau d'une cellule dédifférenciée, de façon à induire un stade de mitose dudit noyau transféré, en ce que l'on prélève ce noyau pendant la mitose et avant la fin de celle-ci, puis l'on introduit ce noyau partiellement dédifférencié en mitose à ce stade dans une cellule, de préférence après en avoir retiré une partie ou la totalité du ou de ses noyau(x), de façon à induire et à terminer sa division nucléaire de différenciation et former une souche cellulaire ou un tissu à un stade de différenciation moins avancé que celui de ladite cellule différenciée.

On peut extraire le noyau transféré au stade de la métaphase, de l'anaphase, de la prophase ou de la télophase de la première mitose.

On peut transférer, dans l'ovocyte, un noyau de cellule myocardique, musculaire ou autorythmique cardiaque, notamment cellules sinusales, du centre de Tawara ou fibres du faisceau de His ou de Purkinje.

On peut soumettre les cellules partiellement dédifférenciées obtenues, de préférence après ou pendant leur culture de multiplication cellulaire après la formation d'un ensemble confluent, à une stimulation périodique électrique du type stimulation cardiaque, notamment selon le procédé de stimulation de l'invention.

De préférence, on soumet lesdites cellules à une stimulation électrique couplée ou pairée dans laquelle cette impulsion sans aucun effet contractile est adressée peu après la fin de la période réfractaire électrique des cellules en culture.

Par exemple, on soumet lesdites cellules à une stimulation électrique selon des cycles comprenant une première impulsion de stimulation et, vers la fin de la période réfractaire, un train d'impulsions, de façon que l'une au moins des impulsions du train tombe peu après la fin de la période réfractaire électrique des cellules et durant leur zone réfractaire mécanique de contraction maximale.

On peut implanter les cellules obtenues par ce procédé au niveau du myocarde auriculaire, notamment dans le cas d'un patient souffrant d'une fibrillation auriculaire.

L'invention a aussi pour objet un segment ou stent artériel, notamment à visée coronaire, aortique, carotide, rénale ou fémorale, comprenant une structure revêtue ou colonisée par des cellules obtenues par les procédés selon l'invention, de préférence, constitué de cellules vivantes, auto-contractiles et élastiques, notamment autologues, cultivées selon le procédé.

Ce segment ou stent artériel peut être conformé à la façon d'un stent artériel agencé pour être introduit dans une lumière artérielle.

De préférence, il comprend des moyens assurant une stimulation électrique de type artériel, coordonnée avec la diastole ventriculaire, desdites cellules du segment, par exemple au moins une électrode de stimulation et/ou de détection.

L'électrode de stimulation et/ou de détection a été, de préférence, introduite dans la culture cellulaire pour être entourée par lesdites cellules vivantes.

Le segment ou stent peut comprendre un capteur, notamment de mesure de saturation d'oxygène et/ou de paramètres métaboliques, et un capteur électrocardiographique de détection ou stimulation.

Il peut présenter une pluralité d'électrodes agencées pour obtenir les variations de l'impédance électrique locale.

De préférence, il comporte une structure, notamment en mailles expansibles supportant des couches cellulaires différentes, telles qu'endoartère, myoartère et périartère, ladite structure permettant une croissance spontanée avec élargissement progressif de sa lumière et création d'une vascularisation nourrissant notamment la partie myoartérielle.

La structure est réalisée, par exemple, en au moins l'un des matériaux suivants : PLGA, collagène, globine, par exemple par tricotage.

On peut, en outre, prévoir un stent artériel sans cellules vivantes possédant une structure radialement expansible, mais assez rigide pour maintenir la lumière artérielle ouverte au moins en systole, éventuellement biodégradable ou retirable par cathéter et susceptible de recevoir un stent physiologique tel que décrit.

Enfin, l'invention a pour objet un pacemaker cardiaque biologique comprenant des cellules ou tissus autorythmiques cardiaques, partiellement dédifférenciées, selon l'une des revendications 110 à 122 ?, de préférence autologue, homologue, provenant de l'organisme receveur, et destiné à être implanté dans le coeur ou une région défaillante du coeur.

L'invention est décrite ci-après en se référant également aux figures annexées dans lesquelles :
- la figure 1 représente schématiquement un électrocardiogramme associé à un mécanogramme du myocarde correspondant au fonctionnement d'un dispositif selon l'invention,
- la figure 2 représente une vue d'un exemple réel,
- la figure 3 représente les acquisitions de différents paramètres dans le fonctionnement de l'invention,
- la figure 4 représente une vue schématique d'un dispositif d'implantation de cellules,
- la figure 5 représente une vue schématique de type synoptique d'un dispositif selon l'invention,
- la figure 6 représente une vue schématique d'un dispositif selon la figure 5, présentant en outre des moyens supplémentaires.
- la figure 7 représente schématiquement les électro-cardiogramme et mécanogramme d'une STIO selon l'invention pour un rythme spontané lent,
- la figure 8 représente schématiquement les électro-cardiogramme et mécanogramme d'une STIO bradycardisante,
- la figure 9 représente schématiquement les différentes zones électriques et mécaniques distinguées dans l'invention,
- la figure 10 représente schématiquement les électro-cardiogrammes et mécanogrammes mis en oeuvre dans la simulation selon l'invention,
- la figure 11 représente un tableau de commande et d'affichage d'un dispositif selon l'invention.

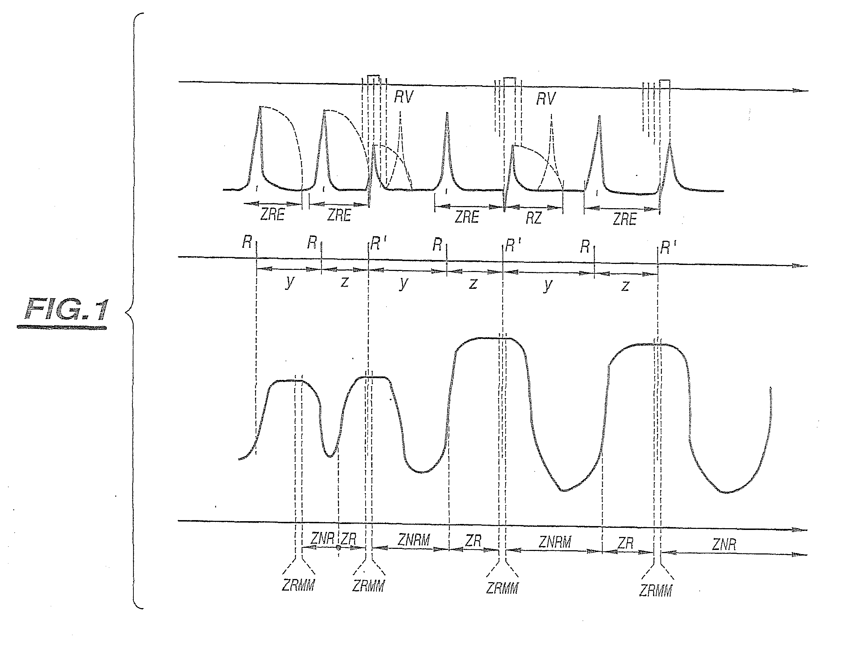

La figure 1 montre un exemple du principe de fonctionnement d'un dispositif selon l'invention. La ligne supérieure montre l'ECG, la ligne médiane montre les couplages Y des salves orthorythmiques par rapport aux ondes R du rythme basal, c'est-à-dire en pourcentage de la durée du cycle antérieur Z et la ligne inférieure montre l'activité mécanique myocardique. Les ondes R pleines représentent les dépolarisations spontanées ou stimulées et les ondes RV en pointillé les ondes R basales inhibées par la prolongation artificielle (RZ) des zones réfractaires induites par les salves des impulsions électriques. Les stimulations en salves de cinq impulsions du stimulateur selon l'invention se voient bien visibles sur l'ECG et la première impulsion de chaque salve, qui provoque la dépolarisation, permet la mesure de chaque zone réfractaire fonctionnelle (ZRE ou RZ) dans chaque cycle. Il faut noter que le début de la zone non-réfractaire électrique (ZNRE) précède le début de la zone non réfractaire mécanique (ZNRM). Sur le mécanogramme, l'intervalle ZRMM correspond à la période de la zone réfractaire systolique contractée maximale encore active durant laquelle une stimulation électrique propagée ne provoque pratiquement ni effet ni dépense énergétique myocardique car ce muscle, se trouvant en contraction maximale juste avant sa relaxation active, est incapable de tout autre action biologique dépensière. La ZRMM est suivie par la zone non réfractaire mécanique (ZNRM). Il faut savoir que tout stimulus électrique survenant immédiatement après la ZRMM, par exemple 20 ms plus tard, tombe en zone ZNRM et peut déjà provoquer une réaction biochimique énergétique précoce de recharge, pour cette diastole ainsi débutante, entraînant une consommation d'oxygène même l'en absence de toute activité mécanique perceptible par les instruments actuels.

L'intervalle ZRM ou ZR correspond à la zone réfractaire mécanique précédant l'étroite zone ZRMM. La zone ZRMM ne correspond qu'à une petite partie du sommet de la courbe des pressions systoliques ventriculaires.

La référence D désigne la diastole électrique.

La description statique ci-dessus des zones fonctionnelles de chaque cycle cardiaque ne montre pas le déroulement dynamique réel au niveau d'un myocarde souffrant. Dans la réalité, les différentes zones fonctionnelles et notamment les ZRE et ZRMM peuvent varier d'un cycle à l'autre, par exemple entre 15 et 80 ms ; ceci en partie déjà favorisé par les médicaments cardio-actifs, le tonus végétatif et par les variations des pré et post-charges, des flux trans-membranaires et d'autres métabolismes intra-cellulaires. On comprend maintenant pourquoi la stimulation pairée classique qui n'utilise qu'une seule impulsion électrique pour prolonger la ZRE, avec un intervalle de couplage constant en ms., ne peut pas réaliser une STIO de sécurité clinique car l'unique stimulus à couplage fixe va tantôt tomber avant, pendant
ou après l'intervalle critique mobile, ce qui reste invisible sur un ECG normal. Il en résulte une stimulation cardiaque physiologiquement inconstante, visible seulement sur ECG spéciaux à grande vitesse de déroulement et sur mécanogrammes intracardiaques très précis (ce qui n'est pas le cas en coronarographie classique) et certaines sollicitations métaboliques, provoquant une surconsommation d'oxygène qui est nuisible pour le myocarde, l'hémodynamique et l'eurythmie et qui est attribué à tort à la seule stimulation pairée.

Durant les cycles STIO traités par l'invention en stimulation pairée ou couplée, peuvent varier non seulement les durées des zones réfractaires, mais également les seuils d'excitabilités. Pour compenser instantanément et continuellement ces irrégularités, il convient de disposer d'un dispositif automatique de réajustement instantané de ces seuils bien connus au niveau des stimulateurs cardiaques implantés et décrits d'abord par l'inventeur dans son brevet français No 1,237,702, P.V. No 651 632 du 11 juillet 1953. Il peut être utile de lancer périodiquement et automatiquement à la demande, selon la fréquence des variations critiques des seuils observés, des salves orthorythmiques dont le voltage varie progressivement ou non et dont les effets sur la dépolarisation électrique provoquée seront automatiquement pris en compte pour réajuster, par exemple, au prochain cycle concerné l'intensité des impulsions composant chaque salve. Cette intensité des impulsions peut d'ailleurs varier régulièrement ou non d'une impulsion à la suivante ou périodiquement à l'intérieur de chaque salve de la STIO. En cas de certaines variations importantes de ces seuils d'excitabilité ou des zones réfractaires, il est souhaitable de contrôler automatiquement si les variations concernent uniquement l'un des paramètres et lequel ou si les deux paramètres varient conjointement et comment. Ces contrôles permettent aussi de réagir préventivement et instantanément en cas d'impossibilité de continuer la stimulation (STIO) selon l'invention telle qu'elle est programmée, par exemple en cas de troubles métaboliques (manque local débutant d'oxygène, tendance aux arythmies, baisse de l'hémodynamique, etc). Dans les cas plus fréquents de STIO qui se maintient suffisamment stable, on peut, par exemple, automatiquement diminuer le nombre, la densité (plusieurs électrodes ou une électrode à grande surface émettant en focalisant sur une électrode réceptrice), la largeur ou l'intensité des impulsions de chaque salve du nouveau pacemaker orthorythmique et inversement les augmenter en cas d'instabilité croissante. On peut obtenir ainsi une exploration automatique de chaque cycle cardiaque adaptée en permanence à ses propres évolutions physiopathologiques permettant de prévenir, de traiter et de signaler instantanément de nombreuses défaillances cardiaques qui, autrement, se révèlent plus ou trop tard. De plus, ce procédé peut permettre de piloter instantanément certaines pompes médicamenteuses implantées (Zacouto, brevet US 5,305,745) et si l'on dispose de plusieurs électrodes, de préférence assez éloignées, de stimulation dans le coeur (par exemple stimulations de resynchronisation ventriculaires) une différence suffisante et subite entre ces zones réfractaires et les seuils d'excitabilité peut indiquer une thrombose coronaire ou lésion myocardique locale. Les fonctions diagnostiques de la STIO selon l'invention peuvent justifier à elles-seules son application, par exemple, en cas d'usage de drogues influençant le fonctionnement cardiaque au besoin en coupant les fonctions de stimulation.

Il convient de distinguer l'usage clinique de la STIO selon qu'elle est appliquée à une tachycardie ou à une insuffisance myocardique. Dans le cas d'une tachycardie mal supportée, rebelle ou récurrente, la STIO permet, dès l'installation des électrodes de stimulations cardiaques, de réduire d'abord rapidement son rythme par environ deux avec une augmentation considérable et immédiate du débit cardiaque, ce qui est dû d'une part à la grande prolongation de la diastole et d'autre part au RPE qui s'y surajoute. Dans certains cas cliniques, notamment tachycardies ventriculaires (TV), il est possible de supprimer ces tachycardies en coupant leurs circuits de réentrées par les salves orthorythmiques réglables de la STIO qui peuvent cibler automatiquement les moindres espaces myocardiques non encore en phase réfractaire lors des dépolarisations ectopiques. Il ne suffit pas toujours de soumettre une TV à la STIO pour la réduire, mais une fois installée, il faut faire varier ses paramètres orthorythmiques par exemple rapprocher les intervalles entre les impulsions, leurs nombres, intensités, largeurs, pourcentage de couplage par rapport à la durée du cycle antérieur, etc. des salves et, au besoin, rapprocher l'électrode de stimulation du lieu de départ des activations ectopiques ; tout ceci peut se faire et se mémoriser automatiquement par exemple par rapport aux spikes de marquage des détections et des stimulations du pacemaker orthorythmique (POR) si l'on dispose de plusieurs électrodes permettant ainsi d'éviter un choc électrique. Dans le cas des insuffisances cardiaques aiguës ou chroniques non provoquées par tachycardies ectopiques rapides, qui sont souvent en tachycardie sinusale modérée, la STIO entraîne immédiatement une diminution du rythme (par exemple de 100 p. min à 60 p. min et une augmentation importante et durable du débit cardiaque et coronaire ainsi qu'une baisse immédiate et persistante des pressions artérielles pulmonaires.

En cas de tachycardies irréductibles ou récurrentes, notamment TV, on peut réduire leur rythme par deux en utilisant une STIO permanente, ce qui peut être une alternative à l'ablation ou permettre d'attendre, sans danger pour le patient, une ablation dans d'excellentes conditions hémodynamiques, ce que même le retour au rythme sinusal ne pourra atteindre. Chez les porteurs d'un défibrillateur automatique implanté (DAI), on peut, en cas de survenue d'une tachycardie dangereuse, résistante ou trop récurrente à la stimulation anti-tachycardique orthorythmique classique, commuter d'abord sur une STIO et seulement si, après environ 10 secondes, une bonne hémodynamique n'est pas rétablie, enclencher le choc de défibrillation ; cette procédure permettra de supprimer de douloureux chocs. Dans le cas des insuffisances cardiaques aiguës ou chroniques, non provoquées par tachycardies ectopiques rapides, qui sont souvent en tachycardie sinusale modérée, la STIO entraîne immédiatement une augmentation importante et durable du débit cardiaque et coronaire ainsi qu'une baisse persistante des pressions artérielles pulmonaires. Un autre avantage de la STIO implantée est qu'elle permet généralement au patient de reprendre une activité physique très augmentée. L'augmentation brusque et importante de l'hémodynamique provoquée par une STIO peut être un inconvénient pour certains patients, dans ces cas on peut programmer la STIO à ne lancer ses salves ou impulsions prolongeant les zones ZRE par exemple, seulement sur une onde R spontanée ou stimulée sur trois ou quatre au lieu de une sur deux ou encore diminuer la durée des diastoles en accélérant le rythme de base. Par exemple, on peut commuter automatiquement le mode de stimulation pairée de la STIO en stimulation couplée, par l'intervention d'un programme imposant une augmentation strictement limitée par un seuil choisi. Une stimulation couplée est une stimulation sans aucune onde R spontanée à rythme totalement imposé par la STIO. Ces deux modes peuvent se faire automatiquement en les pilotant par rapport aux paramètres de l'hémodynamique désirée et de préférence en contrôlant les modifications métaboliques concomitantes tels que la consommation d'oxygène.

Un autre avantage connu des salves d'impulsions orthorythmiques utilisées par la STIO est que sur une salve, par exemple, de 4 impulsions carrées consécutives d'une durée de 0,5 ms chacune sous 1,4 volts avec un intervalle de 15 ms entre les impulsions, si la deuxième impulsion provoque la dépolarisation, les impulsions suivantes vont rapidement dépasser (vitesse de propagation dépassant des Km/sec) une éventuelle fibrillation débutante localement depuis moins de 15 ms (vitesse de propagation environ 70 cm/sec) et l'entourer d'une zone réfractaire ; de plus, la première impulsion de la salve qui tombe en ZRE élimine la propagation de possibles dépolarisations de quelques cellules plus précoces invisibles sur l'ECG, mais pouvant parfois amorcer une arythmie.

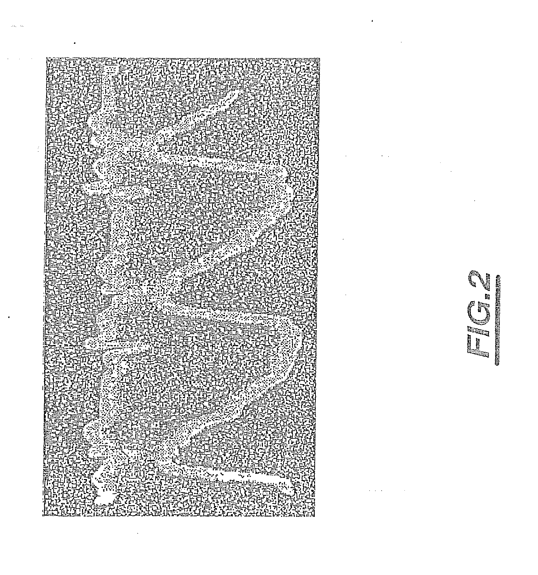

Par ailleurs, la STIO améliore immédiatement le débit coronaire surtout par l'intervention de la RPE et par la diastole très allongée comme cela est connu en coronarographie ainsi que par le début moteur de la diastole qui est accrue proportionnellement au raccourcissement renforcé du myocarde provoqué par la contractilité intensifiée, ce qui correspond à une forte augmentation de la visco-élasticité du myocarde durant les phases motrices ; cette visco-élasticité est mesurable, par exemple, par un détecteur de pression multiaxes implanté dans le myocarde, de tels détecteurs étant connus. La baisse concomitante des pressions artérielles pulmonaires est probablement surtout due à la montée du débit cardiaque. En théorie, la STIO ne devrait pas entraîner une dépense supplémentaire notable d'oxygène. Le ralentissement important du rythme ventriculaire diminue cette consommation d'oxygène alors que l'augmentation de la contractilité, malgré un rendement énergétique amélioré, demande un supplément d'oxygène. Une certaine montée de la consommation d'oxygène au cours de la STIO correspond également à une montée du rapport débit cardiaque sur débit d'oxygène consommé ; la montée subite du débit coronaire permettra le plus souvent de bien tolérer cette dépense supplémentaire d'oxygène. A hémodynamique accrue égale, la STIO consomme moins d'oxygène que les autres rythmes cardiaques. Il existe des rythmes ventriculaires spontanés extrasystoliques à hémodynamique optimale fonctionnant comme une stimulation pairée artificielle comme si la contraction maximale provoque une extrasystole électrique ciblée pour protéger le myocarde d'une prochaine contraction trop rapprochée (fig 2) enregistré par l'inventeur et publié (Dtsch. Gesellsch. Kreislauff. 29 Tagung, p 255-261, 1963 Steinkopff Verlag Darmstadt). Un autre exemple de rythme spontané ressemblant à une stimulation pairée est le découplage électro-mécanique, un QRS sur deux, en cas de tachycardies rapides ; ce phénomène peut perdurer des années et les patients vont souvent bien et sont étonnés lorsqu'on leur révèle leur tachycardie.

La STIO selon l'invention ambitionne de prendre un contrôle optimal du rythme et de la contractilité du coeur, d'une part, en occupant la toute première zone non-réfractaire électrique débutante d'un cycle cardiaque et, d'autre part, en essayant d'assurer une contractilité optimale et un débit sanguin par minute souhaité ; cette dernière prétention nécessite un contrôle en temps réel géométrique et/ou volumétrique et énergétique d'au moins une cavité ventriculaire cardiaque. Dans ce but et pour un POR avec STIO, implantable ou non, il est préférable de prévoir un dispositif exigeant très peu d'énergie, par exemple de type mesure des impédances électriques intra-cardiaques. Le paramètre du déroulement des variations volumétriques est de préférence complété par celui des pressions sanguines intracavitaires et/ou intra-myocardiques concomitantes afin de reproduire une courbe pression-volume correcte, de préférence affichable à l'extérieur du corps par télémétrie classique. Pour un tel stimulateur programmable en automatique, on peut réaliser un dispositif électronique qui intègre, par exemple, la surface des courbes de pression et si possible des volumes ou des vitesses et débits sanguins correspondant (par exemple, par type mesure des impédances électriques intra-cardiaques), par rapport au rythme ventriculaire programmé, et qui impose ensuite un rythme proche de la valeur qui a détecté l'hémodynamique la plus favorable pour un patient donné à un moment donné, après avoir effectué, mémorisé et comparé ces valeurs obtenues après un certain balayage de la fréquence (Zacouto, US 5,306,293). En plus, un réglage automatique des impulsions constituant la salve de stimulation orthorythmique par rapport à l'hémodynamique et/ou à la consommation d'oxygène peut varier le nombre des impulsions, leur intervalle qui peut être non-équidistant, leur largeur, leur forme, leur polarité, leur intensité et leur voltage qui peuvent également être inégaux ainsi que la localisation de leur application au niveau du coeur. Cette localisation de leur application peut comporter des stimulations monopolaires, bipolaires ou multipolaires fixes ou variables, avec électrodes auriculaires ou ventriculaires endocavitaires, intra-myocardiques, épicardiques ou intra-veineux coronaires, intra-artériel coronaires, intra-stents tels que stents spéciaux avec électrodes ECG et/ou de stimulation avec ou sans capteurs hémodynamiques, de saturation d'oxygène et/ou CO₂, pH, glycémie ou autres indicateurs métaboliques. Pour éviter une grande dépense d'énergie de l'électronique de ces appareils implantés, on peut installer une petite antenne HF de part et d'autre de la peau qui transmet une énergie supplémentaire lorsque l'appareil utilise des capteurs et effecteurs thérapeutiques spéciaux.

Le contrôle du rythme cardiaque que donne la STIO selon l'invention comporte la possibilité d'accélérer et aussi de ralentir un coeur accéléré, ce qui le distingue aussi des pacemakers cardiaques classiques. En cas de bradycardie relative induite par STIO, les phases non-réfractaires électriques ne sont pas prolongées autant qu'avec une bradycardie spontanée. Lorsqu'il n'est pas possible avec la stimulation pairée d'obtenir un réglage désiré du rythme ventriculaire accéléré, la STIO peut passer automatiquement en stimulation couplée ventriculaire, ce qui supprime tout complexe QRS spontané et permet d'obtenir un rythme ventriculaire efficace qui est la moitié du rythme ventriculaire rapide stimulé. On peut aussi, si les oreillettes et la conduction hisienne sont normales, comme cela a été réalisé avec succès sur deux patients en insuffisance cardiaque, entraîner les oreillettes à un rythme rapide de l'ordre de 160 p. min et obtenir une STIO ventriculaire vers 80 p. min, ce qui fait persister un QRS ventriculaire entraîné mais quasi-normotrope dans son activation, vu du ventricule.

En dehors des tachycardies régulières, la réduction des phases non-réfractaires dans l'espace myocardique produite par la STIO et ses ajustements donne à cette stimulation cardiaque un effet anti-arythmique notamment pour éliminer les extrasystoles ventriculaires précoces. En cas de fibrillation auriculaire (FA) la STIO ventriculaire pourra permettre de protéger les ventricules des influx de la FA totalement pour les influx de la FA qui tombent précocement dans le cycle ventriculaire (CV) et partiellement pour les influx survenant plus tard dans le CV, si besoin en potentialisant avec certaines drogues ralentissant la conduction hisienne, ce qui redonne aux ventricules un rythme suffisamment régulier, par exemple élimine tous les CV inférieurs à 600 ms., en partie réglable et accompagné d'une contractilité optimale ; ceci peut compenser et surcompenser l'effet défavorable de la FA sur des myocardes fatigués par les tachyarythmies et débits coronaires faibles et potentialiser éventuellement l'effet de la Digoxine et d'autres médicaments cardioactifs.

On peut aussi stimuler un ventricule vers 150 puls./min et obtenir par STIO selon l'invention un rythme efficace régulier vers 75 puls./min bien toléré et à l'abri des influx de la FA. Une STIO auriculaire augmente au maximum la contraction auriculaire et renforce sa puissance musculaire, s'oppose à sa dilatation, aux thromboses, à la réinstallation de certaines FA (fibrillations auriculaires) et améliore les débits ventriculaires et coronaires si les paramètres de la STIO sont bien ajustés par rapport aux activations auriculaires et ventriculaires naturelles ou artificielles. Une stimulation auriculaire peut, par exemple, être réalisée en bipolaire à partir d'électrodes placées sur la moitié supérieure du septum auriculaire, pouvant aider à synchroniser les oreillettes sans gêner les ventricules. Une STIO double, auriculaire et ventriculaire coordonnée, augmente au maximum la contraction auriculaire et ventriculaire coordonnée peut être très utile, avec blocage partiel hisienne ou non, pour accroître considérablement sans délai et longtemps l'hémodynamique cardiaque.

Toute STIO adaptée selon l'invention régénère un fonctionnement génétique à effet inotrope du myocarde avec modifications de l'expression des gènes de développement, des canaux ioniques et des fonctions contractiles induites par les contraintes mécaniques et métaboliques augmentées imposées ; ceux-ci aboutiront à son remodelage intracellulaire de récupération fonctionnelle, par exemple en cas d'insuffisance cardiaque (dilatations) ou de nécroses (ratatinements). Cette thérapie étiologique de chaque insuffisance cardiaque peut donner une involution génétique rapide de son processus pathologique et modification génétique de régénération physiologique et anatomique provoquées par l'effet de la contractilité très augmentée aux possibilités évolutives spécifiques de chaque patient, s'adaptant automatiquement à chaque cas particulier, sauf si certaines maladies telles que métaboliques, tumorales, virales, toxiques etc. empêchent la fonction myocardique de se rétablir. Le maintien du rétablissement de la fonction cardiaque exige une activité musculaire périphérique suffisante qu'il convient de coordonner avec l'action myocardique centrale de la STIO.

Pour certaines nécroses ou fibroses étendues du myocarde, on envisage la greffe autologue locale de cellules souches multipliées et différentiées *in vitro* ou de cellules génétiquement transformées ou reprogrammées par entraînement mécanique électrique et biochimique capables de recoloniser les sites détruits et de métaboliser certains tissus cicatriciels.

On peut procéder à la simple recolonisation locale de cellules isolées partiellement dédifférenciées de myocarde, qui est un syncytium, cellules génétiquement orientées vers des fonctions rythmiques et contractiles avec zones réfractaires, qui peuvent aisément, sous l'effet de l'environnement, s'organiser en réseaux mécaniquement performants bien coordonnés avec le coeur.

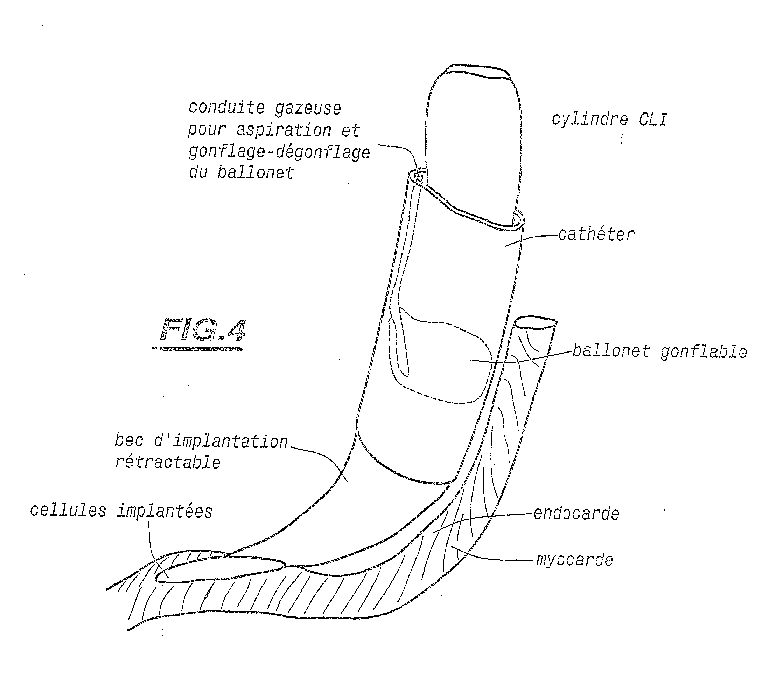

De préférence, on ajoute une programmation préalable de la STIO. La multiplication *in vitro* des cellules à greffer ne doit pas se faire toujours à l'état immobile, mais de préférence aussi, sous une contrainte mécanique alternante contrôlée adaptée aux fonctions futures des cellules et capable d'induire un état membranaire électrophysiologique compatible avec la fonction du coeur à servir, et contrôlée de préférence par une STIO adaptée à la capacité fonctionnelle des cellules afin de les orienter génétiquement vers leur fonction contractile future, et ceci, si possible, avec du sérum autologue. En plus, il convient d'inciter ces cellules à ne pas se multiplier toutes séparément mais, par exemple, à former de petites structures fonctionnelles en trois dimensions en les cultivant, par exemple, sur une matrice préformée, poreuse, élastique et biodégradable tels que PLGA et PLA (dérivés d'acides poly-lactiques) pour éviter leur dissémination après leur injection et favoriser leur fonction contractile en syncytium coordonnée avec la stimulation du coeur. Les petites matrices préformées peuvent avoir des formes 3D qui favorisent leur assemblage mutuel et avec le myocarde du receveur, par exemple sous forme de bandelettes, de disques, d'équerres, de croix, de serpentins, etc. Il convient aussi de faire des analyses électrophysiologiques de ces cellules avant implantation, par exemple à l'aide de micro-électrodes intra-cellulaires pour vérifier les zones réfractaires et les potentiels d'action et de membrane et de contrôler leur capacité motrice par exemple par la mesure des déformations de leur matrice élastique. Ces cellules préparées pour leur fonction contractile peuvent aussi servir en cas de FA à former un tissu myocardique implantable dans les oreillettes, réalisable, par exemple, à travers une voie veineuse cathétérisée, sous contrôle au moins échocardiographique, la ou les sondes pouvant transpercer aussi le septum auriculaire pour ensemencer également l'oreillette gauche ; cette semence sous endocardique d'amas de ces cellules myocardiques implantés soit un à un, soit en pont entre plusieurs enracinements avec précaution pour éviter une perforation de la paroi aussi bien qu'une protrusion détachable dans la cavité auriculaire, ceci peut se faire par un cathéter orientable muni à son extrémité d'un cylindre contenant un étroit bec rétractable du cathéter pointu et souple glissant sur l'endocarde soulevant par aspiration l'endocarde et le piquant très légèrement à cet endroit en poussant un cylindre de cellules à implanter (CLI) dans la petite fissure obtenue à l'aide d'un piston qui est plein en poussée et qui est en recul accolé aux parois du cathéter par exemple sous la forme d'une valve à lèvres élastiques mues par les pressions ou à l'aide d'un ballonnet gonflable et dégonflable capable de couper le CLI, le recul ou dégonflage faisant monter un nouveau « cylindre » de cellules, de longueur ajustable de l'extérieur, devant le piston ou ballonnet dans le cathéter par pression d'un fluide (fig. 4). Les amas et ponts de cellules implantées (CLI) vont s'étendre et former un réseau à mailles contractiles pilotées par une STIO ou stimulation simple, par exemple située dans l'oreillette droite et les CLI devraient, par effet de proximité, fusionner dans le syncytium d'origine et y induire certaines parties de leur équipement génétique. Un tel cathéter d'implantation cellulaire peut s'appliquer à toute région du coeur et d'autres organes tels que reins, pancréas, foie etc. Ce principe d'implantation de cellules autologues multipliées et pouvant se multiplier facilement et préparées pour une fonction contractile peut aussi s'appliquer à la construction d'un coeur artificiel total ou partiel constitué au moins partiellement par des cellules autologues cultivées, de préférence à partir de cellules myocardiques partiellement dédifférenciées.

Dans une réalisation préférée de l'invention, on réalise un clonage thérapeutique cellulaire partiel qui consiste à introduire dans un ovocyte dénucléé un noyau étranger total ou partiel et de retirer ce matériel nucléaire à un stade voulu non achevé de sa mitose et de l'implanter ensuite dans une cellule de préférence autologue, différenciée dénoyautée ou non, dans laquelle se terminera la première mitose conduisant à une dédifférenciation partielle.

On peut ainsi envisager un clonage partiel purement nucléaire consistant à retirer le noyau cellulaire en mitose sélectionnée et non achevée de l'ovocyte avant la première division cellulaire complète, à un moment déterminé, par analyse par exemple optique, de l'évolution du noyau. Ainsi, par exemple, durant la prométaphase, si une dédifférenciation légère est souhaitée ou, durant l'anaphase ou la télophase, si une différenciation plus forte est souhaitée. Ensuite, on l'introduira dans une membrane cellulaire vivante de préférence vidée de son noyau, de préférence autologue, embryonnaire ou de cellules souches par exemple dermique, épithéliale, lymphatique, conjonctive, osseuse, cartilagineuse, sanguine ou musculaire. On pourra ensuite introduire ces cellules, résultant d'un clonage inachevé, dans une cellule myocardique où elle pourra exercer une transcription et expression génétique régénératrice capable d'induire au moins localement et temporairement, dans son environnement, une action et contamination génétique partielle d'un degré souhaité.

On peut aussi, dans une variante de l'invention, introduire dans l'ovocyte un noyau se trouvant déjà, spontanément ou par provocation artificielle connue, en mitose non achevée voire en mitose achevée, sous forme des deux premières cellules, afin d'obtenir plus aisément une dédifférenciation partielle.

Etant donné la rareté des ovocytes humains ou de mammifères, on envisage de rendre un ovocyte multipare en lui introduisant un nouveau noyau après avoir retiré délicatement le premier noyau en mitose partielle. Cette multiparité par inoculation intra-ovocytaire consécutive peut avoir sur le matériel nucléaire un effet de dédifférenciation différent de celui obtenu lors de la première introduction du noyau ou des chromosomes ou gènes de ce même ovocyte.

On peut également introduire dans l'ovocyte un noyau, qui soit par exemple déjà en mitose spontanée ou provoquée partielle, ou par ailleurs des chromosomes ou gènes ou partie de noyaux à traiter dans une cellule de type embryonnaire. Celle-ci sera de préférence du même tissu, par exemple myocardique, de préférence partiellement dénoyauté et cultivable *in vitro*, *vivo* ou *in situ*. Cette ou ces cellules seront cultivées pour multiplication de préférence un temps suffisant in vivo dans des tissus embryonnaires pour obtenir une dédifférenciation partielle. Les noyaux ainsi traités peuvent être laissés, soit dans les cellules de type embryonnaire pour constituer un tissu greffable dans l'organisme de provenance du noyau, soit extraits de leur cellules hébergentes pour devenir inducteur de régénération cellulaire locale au niveau d'un tissu différencié, de préférence autologue et identique. L'implantation du noyau ou parties nucléaires peut également se faire à l'intérieur d'une cellule souche, de préférence de type embryonnaire ou foetal.

De telles cellules partiellement et sélectivement dédifférenciées peuvent ensuite être introduites dans des cellules myocardiques, de préférence d'origine, en survie, de préférence préalablement plus ou moins dénoyautées et servir de tissu myocardique régénérant pour être implantées, par exemple, dans des régions sclérosées par fibrose, mal vascularisées, ou à contractilité insuffisante du coeur.

A partir d'un certain degré de dédifférenciation, ces cellules perdent leur pouvoir immunogène et peuvent être utilisées pour régénérer des tissus myocardiques non autologues.

Cette fonction comporte aussi la capacité de ces cellules génétiquement activées d'agir à distance par sécrétion, libération ou induction, notamment par molécules biochimiques spécifiques. Cette activation génétique trans-humorale montre, entre autres, la capacité de mobiliser des cellules pro-génératrices d'apparition locale tels qu'on les observe dans des infarctus myocardiques étendus.

Cette préparation cellulaire, applicable aussi à d'autres types cellulaires, peut créer des tissus de régénération contrôlée permettant de soigner de nombreuses lésions organiques et tissulaires. Ainsi, par exemple, en prélevant, sous contrôle échographique, par une aiguille trans-rectale des cellules prostatiques qui seront totalement ou partiellement clonées et en les réinjectant par la même voie dans la prostate, ce rajeunissement cellulaire inducteur pourra, dans certains cas, s'opposer au développement d'un cancer local ou freiner son extension. Ainsi aussi, une rétine ophtalmique autologue, voire homologue, régénérée pourra avoir un grand intérêt en cas de DMLA, et l'insuffisance rénale grave pourra être combattue par l'implantation de cellules partiellement dédifférenciées obtenues, après transfert dans puis hors d'ovocytes, de noyaux de différentes cellules de néphron, et l'arthrose soulagée par l'implantation de chondrocytes provenant d'un clonage partiel ; il en va de même pour des surfaces cutanées et notamment pour régénérer une chevelure normale, par exemple en transférant dans l'ovocyte un ou des noyaux ou parties de noyaux de cellules de follicule pileux et/ou de mélanocyte, pour une régénération de la chevelure et/ou de sa couleur.

Une application importante de l'invention consiste à renforcer ou recréer les fonctions thymiques par rajeunissement génétique de cellules thymiques homologues ou si possible autologues partiellement dédifférenciées pour réanimer activement les fonctions immuno-protectrices du corps.

En cas de fibrillation auriculaire, une application principale de l'invention consiste à transplanter ces cellules partiellement dédifférenciées et fonctionnellement entraînées à la contraction synchronisée en laboratoire en les implantant, par exemple, en nappes ou en grilles en contact du myocarde auriculaire où elles peuvent progressivement se multiplier et/ou croître et/ou génétiquement modifier les cellules malades du myocarde auriculaire, et/ou encore aussi agir par activité de sécrétion et induction humorale, biochimique, mécanique ou physique, peut être en intervenant dans un champ morphogénétique.

Dans une forme de réalisation avantageuse, on excise sous microscope une cellule myocardique et on garde la membrane et le cytoplasme vivant en culture après avoir extrait un, plusieurs ou tous les noyaux. Ce ou ces noyaux extraits seront introduits isolément ou en groupe dans un ovocyte ou plusieurs ovocytes de préférence préalablement énucléés, et on attendra, de préférence par observation sous microscope, seulement la pro-, méta-, ana-, ou téléphase avant de les prélever et de les réintroduire dans la membrane et le cytoplasme conservé vivant ou vivable en culture ou bien dans une autre cellule syncitiale myocardique, de préférence vidée d'une partie ou de tous de ses noyaux.

Ces cellules à noyaux plus ou moins dédifférenciés seront mis en culture appropriée et multipliées, de préférence sur des tuteurs élastique biodégradables, par exemple une matrice de collagène ou un textile élastique biodégradable *in vivo*, et stimulées de préférence progressivement par pacemaker orthorythmique simple ou inotrope afin de s'adapter physiologiquement à leur insertion future, soit dans le myocarde malade ou sénile du malade, soit pour constituer les parois auto-contractiles d'un coeur artificiel partiel ou total, et implantées ultérieurement chez le patient, notamment aussi au niveau auriculaire pour supprimer une fibrillation auriculaire, notamment aussi pour former des segments d'artères coronaires actives et contractiles utilisables comme stents coronaires conducteurs et propulseurs physiologiques, cette contractilité coronaire et distensibilité active et passive complémentaire pouvant être provoquée au besoin par une stimulation artérielle électrique du muscle lisse artériel décalée en diastole par rapport à la contraction ventriculaire, ou former une artère coronaire presque entière ou supplémentaire, par exemple, pouvant provenir d'une dédifférenciation ou clonage partiel de cellules artérielles autologues musculaires et aussi endothéliales coronaires reconstituant une artère coronaire autologue rajeunie fonctionnelle sur éventuellement des tuteurs élastiques ou extensibles, résorbables ou non.

Dans une variante de l'invention, on introduit une partie sélectionnée d'un chromosome ou un chromosome entier ou gène dans un ovocyte fonctionnel convenable, soit isolément, soit accompagné d'une partie du noyau ou du noyau cellulaire différencié, de préférence autologues d'origine ou non d'origine, partiels ou entier à traiter. Ces noyaux à traiter peuvent être privés sélectivement du ou des chromosomes malades qu'il s'agit de remplacer afin de faire tolérer par le noyau sa reconstitution chromosomique génétique de dédifférenciation sélective et/ou partielle.

Bien entendu, on peut substituer à la partie de chromosomes ou aux chromosomes entiers à dédifférencier un ou un groupe de gènes sans pour autant franchir le domaine de l'invention.

On peut ainsi transplanter un ou des groupes de gènes dans un ovocyte adapté, en présence ou non d'un ensemble sélectionné de chromosomes ou d'au moins un noyau cellulaire dont la dédifférenciation sélective et partielle, par exemple en métaphase ou anaphase, permettra par observation microscopique optique à distance, de surveiller précisément les réorganisations chromosomiques de la préparation de la division cellulaire permettant l'isolement des chromosomes et la destruction sélective d'un ou plusieurs chromosomes ou régions chromosomiques, par exemple par rayonnement laser, d'une façon en soi connue, et leur remplacement par un ou des chromosomes ou régions correspondants sains, et de soumettre ensuite le noyau ainsi modifié à une dédifférenciation partielle dans un ovocyte.

L'extraction des noyaux ou chromosomes partiellement dédifférenciés pourra se faire, par exemple, par injection intra-ovocytaire d'un sérum adapté sous pression légère après avoir suffisamment mobilisé le matériel nucléaire avec une micro-tige ou palette ou par l'action vibratoire d'ultrasons ou de laser.

On pourra aisément vérifier par le degré d'intégration nucléaire ou péri-nucléaire, cette intégration étant optiquement visible.

En cas de création de chromosomes ou gènes artificiels entier ou partiels ou artificiellement induits, il convient souvent de soumettre ces éléments à une telle dédifférenciation partielle afin de leur créer un passé d'évolution génétique sans lequel leur avenir peut-être compromis. Lorsque ces éléments artificiels à fonction génétique ne supportent aucune involution génétique de dédifférenciation, leur fonctionnalité future est incertaine.

Les greffes tissulaires nécessitent pour leur succès l'élimination suffisante des réactions lymphocytaires de rejets graves généralement de type antigène, anticorps et humoral de l'organisme receveur tel qu'observé chez la femme enceinte qui tolère biologiquement, à travers son placenta, son foetus. Il convient, par exemple, d'imiter génétiquement et/ou plasmatiquement ces caractéristiques de tolérance, notamment lymphocytaires et des fonctions du thymus. Chez l'être humain adulte et surtout âgé, la présence des cellules et fonctions du thymus est souvent trop faible voire absente. Il peut être utile, par exemple, de transplanter un tissu de thymus homologue, partiellement et sélectivement dédifférencié et génétiquement adapté, par exemple, par intégration chromosomique sélective de chromosomes du greffon dans des noyaux cellulaires de l'organisme receveur et ceci soit par préparation génétique de cellules souches sanguines de l'organisme receveur soit par préparation en culture cellulaire in vitro ou in vivo (tels que sous- ou intradermique) avec ou sans hyper-expression de leur télomérase ou protéine Sir2 pour prolonger leur vie et pour provoquer leur accoutumance induite aux antigène et anticorps critiques des cellules à greffer au moyen connus de l'influence induite à distance par des substances biochimiques et hormonales sur les expressions de certains gènes.

De telles cellules génétiquement adaptées à différents tissus receveurs sont notamment utilisables pour la cure radicale de la fibrillation auriculaire ainsi que pour la régénération rétinienne, les implantations de tissus pancréatiques sans membrane de protection, de néphrons régénérants, de cellules dopaminergiques intra-cérébrales contre la maladie de Parkinson, de tissus de régénération dermique et épidermique, y compris chevelure, de tissus prostatiques ou de glandes mammaires pour la cure des cancers correspondants, et notamment de cellules de thymus de régénération et toute autre cellule à fonctionnement utiles ou indispensables notamment des tubes et systèmes digestifs, nerveux, dermiques, respiratoires, squelettiques et cardiovasculaires.

Les lésions artérielles coronaires, carotides, rénales, etc., peuvent être mortelles et nécessitent idéalement un traitement radical de régénération tissulaire et fonctionnelle. Dans ce but, l'invention comporte une variante capable de prélever des cellules musculaires artérielles autologues ou homologues et de les soumettre à une dédifférenciation partielle contrôlée selon les méthodes susmentionnées. On peut ensuite cultiver ces cellules artérielles soit *in vitro* autour d'un tuteur de préférence élastique, dissolvable ou non, en forme de tube et dans un sérum circulant intra-tube sous pression pulsatile augmentant progressivement afin de recréer la fonction contractile et de relaxation élastique des cellules afin de reconstituer un tube artériel au diamètre voulu. On cultivera de même des cellules endothéliales artérielles partiellement dédifférenciées sous forme d'un tube de plus petit diamètre que l'on introduira à la fin dans le plus grand tube ainsi cultivé. De tels segments artériels partiellement dédifférenciés et tolérés par l'organisme pourront ensuite soit remplacer des segments artériels malades soit s'implanter en parallèle ou en supplément d'un réseau défaillant.

A l'échelle des artères coronaires, on peut ainsi créer, par exemple, des « stents » vivants et auto-contractiles, implantables par exemple par cathétérisme endo-artériel. Les stents actuels sont faits dans une matière plastique ou métallique inerte et immobile et remplacent un segment artériel plus ou moins auto-contractile mais thrombosé, contribuant à la désertification des petites artères et capillaires coronaires en aval. Ces stents artificiels non seulement n'ont aucune action active telle que propulsion sanguine coordonnée avec l'onde artérielle locale motrice mais encore perturbent la dynamique de l'écoulement sanguin normal de toute l'artère concernée, entraînant souvent une désertification des petits vaisseaux en aval des stents.

Par contre, un segment d'artère coronaire physiologique reconstitué, autologue, vivant, pulsant activement en renforçant l'ondée artérielle et au besoin génétiquement « rajeuni » par dédifférenciation partielle ne provoquant pas de réactions de rejet de l'organisme receveur de la greffe, peut reconstituer un segment artériel physiologique très actif qui peut être capable, en plus, d'améliorer la circulation sanguine de toute l'artère, par exemple par sa capacité mécanique (sollicitant la sensibilité mécanique de certaines expressions génétiques) et par sécrétions et induction génétique de facteurs pouvant provoquer à distance un certain rajeunissement cellulaire.

Normalement, ce « stent physiologique pulsatile » sera activé spontanément, notamment par les variations des pressions pariétales et sanguines. Si besoin, l'activité rythmique coordonnée de ce segment de « stent physiologique » pourra être assurée par un pacemaker électronique artériel adapté au rythme du battement artériel, par exemple par un détecteur des pressions intracavitaires ventriculaires ou intramyocardiques et/ou l'ECG et stimulant en diastole, par exemple, aussi par rapport au rythme du pacemaker cardiaque.

On peut aussi placer sur un tel stent artériel, de préférence vers son extrémité distale, un capteur des pressions, de la saturation d'oxygène et de l'ECG, au cours de la reconstruction tissulaire *in vitro*, ces capteurs étant ainsi entourés totalement par du tissu vivant, et relier les fils de sortie, de préférence au pacemaker cardiaque ou défibrillateur implanté, ce qui permet de connaître en permanence la circulation sanguine locale efficace sans pratiquer une radioscopie momentanée avec injection de substance de contraste.

Afin de préserver les besoins d'une intervention sans délai d'une pose de stent classique, il convient d'implanter en urgence un stent immobile qui soit progressivement dissolvable ou retirable ou infiltrable par le « stent physiologique de réactivation coronaire » par cathétérisme.

Dans une variante préférée de l'invention, ce segment artériel coronaire reconstitué est pourvu, en continuité vivante, d'artères, d'artérioles, de réseaux capillaires et veineux avec leurs tissus myocardiques irrigués ; ce bloc tissulaire fonctionnel étant préalablement cultivé et multiplié *in vitro* ou *in vivo*, de préférence à partir de cellules autologues, partiellement dédifférenciées, un abouchement veineux suffisant de ce tissu étant prévu vers la cavité ventriculaire ou le sinus veineux coronaire. Ce bloc myocardique rénové très fonctionnel pourra induire progressivement une recolonisation des capillaires et petites artères coronaires du myocarde originel.

L'implantation d'un stent physiologique par cathétérisme artériel classique fonctionnera au début par alimentation sanguine artérielle d'imbibition, comme réalisée durant sa mise en culture, à partir de l'endo-artère.

Progressivement, la grande vitalité du stent physiologique rénové créera de nouveaux vaso-vasorum. Ultérieurement, la vitalité dominante de ce segment artériel produira un certain élargissement du calibre de ce stent en fonction de ses besoins métaboliques.

Une application supplémentaire de l'invention consiste à reconstituer progressivement *in vitro*, de préférence après dédifférenciation partielle des premières cellules des différents tissus cardiaques et leur multiplication débutante, une partie de ventricule, par exemple en cas d'anévrisme ou de nécrose ventriculaires, ou l'ensemble de deux ventricules, en utilisant des tuteurs extensibles ou assez souples ou biodégradables et les facteurs de croissance adéquats et en respectant la géométrie ventriculaire en trois dimensions assurant les connexions vasculaires telle que l'on peut l'obtenir à partir d'une échographie cardiaque en trois dimensions (N. Mirochnik, A. Hagège, F. Zacouto et C. Guérot : Arch. Mal. Coeur, Paris, 93, 10 octobre 2000).

Dans d'autres variantes, l'invention peut s'appliquer aux greffes cellulaires et tissulaires sans réactions graves de rejets, par l'utilisation de clonages partiels et/ou sélectifs.

Dans une variante de l'invention, on introduit une partie sélectionnée d'un chromosome ou un chromosome entier dans un ovocyte fonctionnel convenable soit isolément soit accompagné d'un ou de deux noyaux cellulaires, de préférence autologues du receveur ou non d'origine, partiels ou entiers à traiter. Ces noyaux à traiter peuvent être privés sélectivement du ou des chromosomes malades notamment isolables au cours d'une phase de leur mitose qu'il s'agit de remplacer par des chromosomes équivalents de préférence de types embryonnaires ou génétiquement partiellement dédifférenciés afin de faire tolérer par le noyau sa reconstitution chromosomique génétique de dédifférenciation sélective et/ou partielle.

Bien entendu, on peut substituer à la partie de chromosomes ou aux chromosomes entiers à dédifférencier un ou un groupe de gènes sans pour autant franchir le domaine de l'invention.

On peut ainsi transplanter un ou des groupes de gênes dans un ovocyte adapté, en présence ou non d'un ensemble sélectionné de chromosomes ou d'au moins un noyau cellulaire dont la dédifférenciation sélective et partielle, par exemple en métaphase ou anaphase, permettra par observation microscopique optique à distance, de surveiller précisément les réorganisations chromosomiques de préparation de la division cellulaire à l'aide des mouvements et apparitions des éléments intracellulaires ou nucléaires centriolaires, le début de la formation d'une membrane nucléaire, mouvements et densification des chromosomes, etc. En d'autres termes, il s'agit de dédifférencier suffisamment soit des groupes de gènes, soit des chromosomes sélectionnés au préalable et de les transplanter ensuite dans un ovocyte porteur du noyau devant être génétiquement modifié et préalablement dépourvu du ou des chromosomes malades concernés.

Une variante de l'invention consiste à co-dédifférencier partiellement dans un même ou autologue ovocyte dénucléé pour induire une tolérance immunitaire réciproque entre, d'une part, un noyau cellulaire dont un ou plusieurs chromosomes ou parties de chromosomes malades ou séniles ont été détruits lors de sa mitose et, d'autre part, une ou plusieurs parties de chromosomes ou de chromosomes entiers sains, de préférence homologues, sains et plus jeunes. De telles cellules génétiquement réparées et dédifférenciées peuvent aussi recevoir une surexpression de leur télomérase ou protéine Sir2.

L'extraction des noyaux ou chromosomes partiellement dédifférenciés pourra se faire, par exemple, par injection intra-ovocytaire d'un sérum adapté sous pression légère après avoir suffisamment mobilisé le matériel nucléaire avec une micro-tige ou palette et/ou l'application d'ultrasons et/ou de lasers locaux à effet vibratoire.

On pourra aisément vérifier le degré d'intégration nucléaire, cette intégration étant optiquement visible.

En cas de création de chromosomes ou gènes artificiels entier ou partiels ou artificiellement induits ou disposés, il convient souvent de soumettre ces éléments à une telle dédifférenciation partielle afin de leur créer un passé d'évolution génétique sans lequel un avenir peut-être compromis. Lorsque ces éléments artificiels ou artificiellement combinés, à fonction génétique, ne supportent aucun passé par dédifférenciation génétique ciblée, leur fonctionnalité future est incertaine.

Un exemple de mise en route du procédé peut être d'acquérir l'hémodynamique et éventuellement la consommation d'oxygène correspondante et de déterminer ensuite les intervalles de couplages de la stimulation pairée, soit par rapport à l'onde R de l'ECG en ms, soit par rapport à l'intervalle R-R en pourcentage de la durée du cycle antérieur, soit par une combinaison algorythmique des deux. On envoie un train d'impulsions pour, au moins, un cycle. On acquiert l'hémodynamique pour un cycle et on la compare à l'hémodynamique spontanée en vérifiant que l'augmentation obtenue dépasse un pourcentage programmé, par exemple 20%. Si on atteint des valeurs voulues d'hémodynamique et éventuellement de consommation d'oxygène, on continue cette STIO et on continue l'acquisition de l'hémodynamique et préférentiellement de la consommation d'oxygène myocardique. Si on n'atteint pas les valeurs programmées, on modifie, au moins, un paramètre des trains d'impulsions et on refait les contrôles. Si, après les modifications successives programmées des paramètres des trains d'impulsions, on n'obtient pas les valeurs voulues, on arrête la STIO.

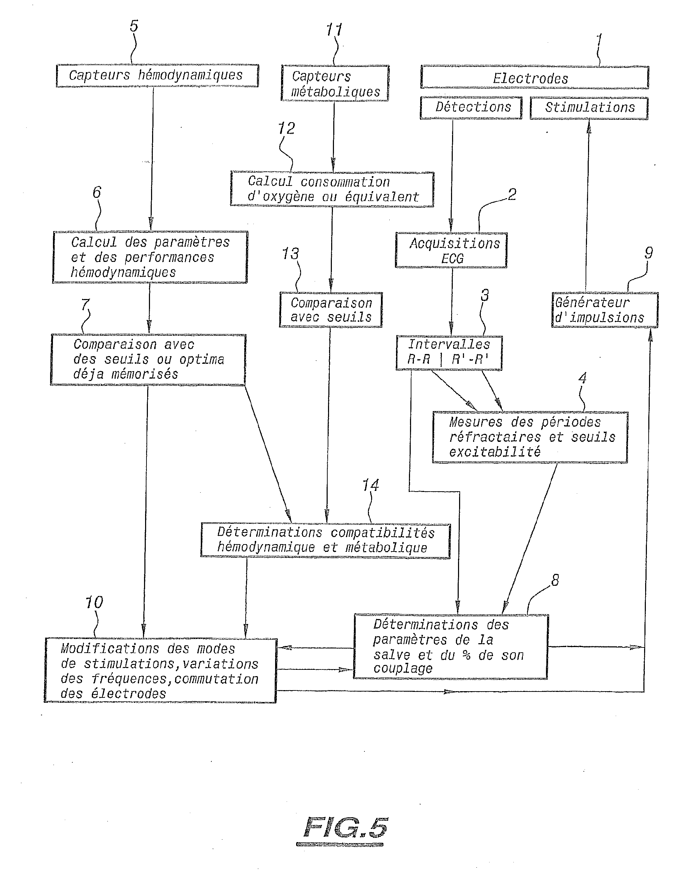

A titre d'exemple, on a représenté, sur la figure 5, une vue synoptique d'un dispositif implanté selon l'invention.

La réalisation technique des différents composants matériels ou logiques ne sera pas détaillée davantage, qu'il s'agisse des moyens de détection ou de stimulation, des sources d'énergie, et des moyens de traitement logique et de mémoire, qui sont tous maintenant tout à fait classiques et bien connus dans les stimulateurs implantés.

Le dispositif comprend des moyens de détection et de stimulation électrique 1. Par exemple, des électrodes de détection qui peuvent d'ailleurs aussi être utilisées pour la stimulation, comme cela est souvent le cas. Des moyens de détection alimentent en signaux des moyens d'acquisition de l'électrocardiogramme 2. Ces moyens d'acquisition permettent d'obtenir notamment, et de mémoriser, dans des moyens logiques 3, le rythme cardiaque, à savoir les intervalles RR des ondes génératrices des contractions musculaires et R-R', c'est-à-dire, l'intervalle entre l'onde R et une onde R' couplée provoquée conformément à l'invention à la fin de la période réfractaire (K. Theisen, F. Zacouto, M. Grohmann, H. Jahrmärker : Refraktärzeitmessung bei absoluter Arrythmie mit orthorythmischer Serienstimulation, Klin. Wochenschr., 52, 1082-1084 (1974), Springer-Verlag). Ces moyens permettent également la détermination de celle des impulsions de la salve d'impulsion qui a provoqué l'onde R'.

Il est ainsi possible d'obtenir en 4 la détermination de la période réfractaire.

Le dispositif comporte également des moyens de détection de l'hémodynamique 5, tels qu'un capteur de pression intracardiaque ou intramyocardique et des capteurs de volume par mesure de l'impédance, et des moyens mesurant l'énergie cinétique de chaque volume expulsé (par exemple en mesurant la pente Δp/Δt des variations de pression et/ou de volume, en encore par des capteurs implantés à effet Doppler ou des accéléromètres), ces moyens permettant, dans des moyens 6, l'acquisition de ces données le calcul de la performance hémodynamique, c'est-à-dire du volume de sang éjecté et, en le rapportant au rythme, du débit cardiaque. De préférence, les capteurs hémodynamiques 5 comprennent un capteur de pression intramyocardique situé près d'une électrode intraventriculaire de détection et de stimulation faisant partie des moyens 1. Les moyens 6 sont sensibles à l'amplitude de la pression détectée par le capteur de pression, et vérifient si cette amplitude varie peu après la montée en pression, pour déterminer la zone ZMRR. Les valeurs provenant des moyens 6 sont adressées à des moyens de comparaison 7 dans lesquels on a également mémorisé soit un seuil au-dessus duquel on souhaiterait maintenir l'hémodynamique, soit un optimum hémodynamique qui aurait été enregistré, par exemple antérieurement, par le dispositif selon l'invention. Ces moyens 7 adressent le résultat de leur calcul à des moyens 8 qui reçoivent également les informations relatives à la période réfractaire provenant des moyens 4 et aux caractéristiques de l'électrocardiogramme, notamment le rythme provenant des moyens 3. Dans les moyens logiques 8, on détermine le couplage, c'est-à-dire, l'intervalle que l'on prévoit entre la dernière onde R qui vient d'être détectée et l'envoi de la stimulation ou de la salve d'impulsion de stimulation, ces moyens peuvent également, le cas échéant, modifier, en fonction des informations reçues, non seulement l'intervalle de couplage s'il y a lieu, mais également d'autres caractéristiques, telles que la durée de la salve, le nombre d'impulsions, l'intervalle entre les impulsions ou encore l'intensité ou la durée de chacune des impulsions.

Les moyens 8 pilotent des moyens 9 de génération de salve d'impulsion.

Les moyens de comparaison 7, en fonction du degré d'efficacité de l'hémodynamique constatée et de sa comparaison par rapport à des valeurs voulues, peuvent encore, éventuellement, modifier la fréquence des stimulations par des moyens 10 pilotant le générateur d'impulsions 9, par exemple notamment, dans le cas où l'électrogénèse spontanée du patient est insuffisante à provoquer un rythme cardiaque convenable.

Le dispositif peut encore comprendre des capteurs métaboliques 11, par exemple de pression de saturation d'oxygène local ou de son équivalent en concentration d'électrons transmembranaires ou libres tel que mesurable par un coefficient d'oxydo-réduction à l'intérieur d'une veine du sinus coronaire et, par exemple, relatif au sang veineux intra-auriculaire droit et artériel intra-auriculaire gauche et/ou des capteurs de CO₂, et/ou de pH etc. reliés à des moyens d'acquisition 12 déterminant une valeur, telle que la consommation d'O₂ acquise pour une comparaison dans des moyens 13 avec un ou des seuils programmés ou antérieurement mémorisés. On peut, ainsi, mettre en oeuvre un moyen logique 14 qui influe sur le moyen 10 (ou dans un cas plus simple, sur le moyen 8) pour modifier s'il y a lieu les paramètres ou le couplage de la salve, ou d'autres variations prévues dans les moyens 10.

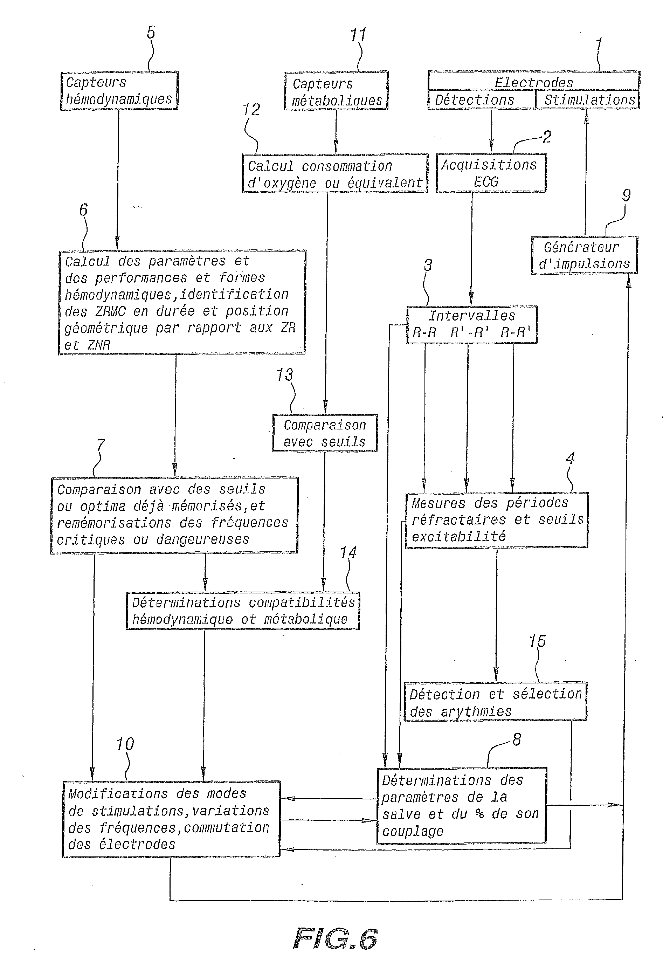

Le dispositif représenté sur la figure 6, présente les moyens identiques à celui décrit pour la revendication 5, et des moyens supplémentaires. Ainsi le moyen 6 est agencé pour pouvoir identifier les zones ZRMM en durée et en position géométrique par rapport aux zones ZR et ZN R.

Les moyens de comparaison avec des séries optimums déjà mémorisées peuvent être agencés pour mémoriser des séquences critiques dangereuses ayant déjà existé ou entrées par programmation et permettre une action en cas de détection d'une séquence de cycles que l'on estime critique, pour, par exemple agir sur un moyen de défibrillation ou pour stopper le fonctionnement du dispositif selon l'invention et le faire fonctionner à la façon d'un pacemaker orthorythmique classique ou VVI ou DDD.

Le moyen 3 est agencé également pour acquérir également les intervalles R-R' de façon directe.

Enfin, un moyen supplémentaire 15 est disposé pour recevoir des informations depuis les moyens 4, ces derniers étant agencés pour acquérir des informations également sur les arythmies, ledit moyen 15 permettant ainsi la détection et la sélection des arythmies, de préférence la distinction entre les arythmies spontanées et celles induites par le dispositif, lesdits moyens 15 rétroagissant sur le moyen 10, qui commande l'envoi des impulsions.

En échocardiographie, la STIO permet au repos de faire immédiatement suivre à un CC basale un ou plusieurs CC à contractilité maximale ou inversement et mesurer leur différence quantitative et géométrique. Pour une épreuve d'effort progressive, il faudra cependant utiliser les moyens classiques. La comparaison entre la contractilité maximale de la STIO et celle obtenue par effort physique ou substances du type Dopamine, Noradrénaline, etc., pourra donner de nouvelles indications sur la fonction myocardique.

La mesure automatique des variations des zones réfractaires ZRE et ZRM durant des épreuves d'effort, notamment sous contrôle échocardiographique, permettra de connaître un état myocardique intracellulaire.

En cas de stimulation cardiaque bi-ventriculaire de resynchronisation fonctionnelle utilisée, par exemple, en cas de bloc de branche gauche, d'hypertrophie grave du myocarde, etc. on peut, en principe, améliorer encore le résultat hémodynamique en ajoutant une STIO pilotée par le stimulateur bi-ventriculaire qui renforcera la contractilité intrinsèque de chaque ventricule et conduira à une régénération génétique que l'on observe au niveau des myocardes soumis à un entraînement physique intensif prolongé avec exercices musculaires périphériques.

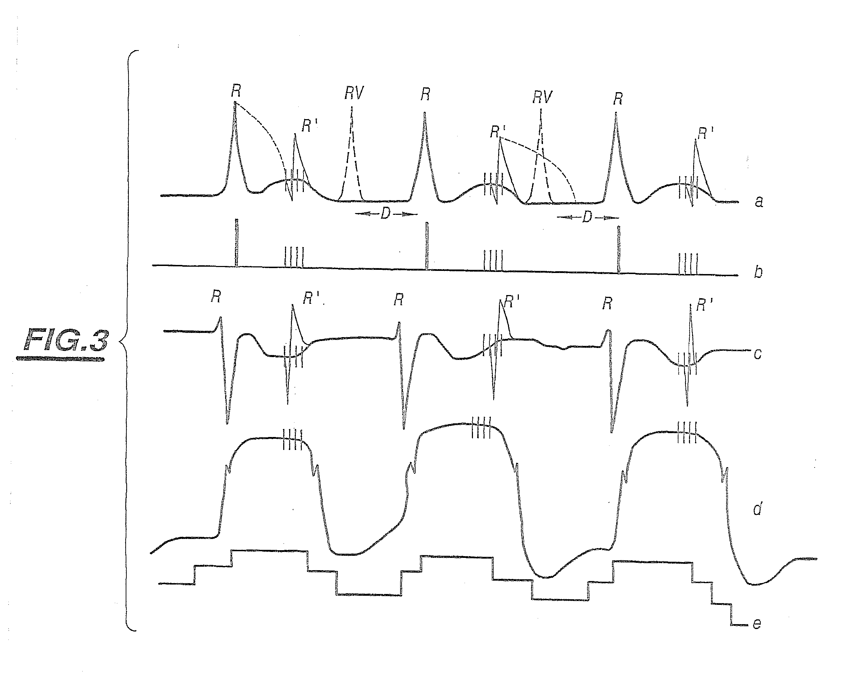

Une STIO automatique selon l'invention comporte un dispositif permettant d'assurer une hémodynamique optimale dans des limites réglables par exemple de rythme, de pressions : max., min., différentielle, de variations dimensionnelles de la contraction, saturations locales et générales d'oxygène (de préférence dans le sinus coronaire et le sang artériel et veineux), de continuité régulière de la STIO, etc. Afin de pouvoir réaliser cette mise au point, il convient de disposer par exemple (fig. 3) d'un affichage spécialement programmé au niveau d'un oscilloscope à plusieurs voies simultanées et en utilisant les traitements de signaux et algorithmes connus, qui comporte par exemple, en temps réel, les paramètres suivants :
a) un tracé d'ECG montrant 3 à 5 cycles à déroulement rapide,
b) le marquage des spots (spikes) de détections et de stimulations au niveau des électrodes sur une ligne,
c) un tracé montrant l'ECG intra-cardiaque de préférence en mono-polaire, par exemple sur une sonde tri-polaire, la stimulation se faisant en bi-polaire, ce qui permet de déterminer laquelle des impulsions d'une salve entraîne effectivement la dépolarisation électrique propagée permettant ainsi la mesure automatique des ZRE et de leurs variations à chaque CC, mesures rendues possible par le pacemaker orthorythmique, même en cas de fibrillation auriculaire (K. Theissen, F. Zacouto, Klin. Wschr. 52, 1082-1048, 1974, Allemagne, Springer-Verlag),
d) un tracé montrant les courbes mécaniques, variations des pressions, volumes et accélération,
e) un tracé montrant la consommation d'oxygène ou un équivalent si possible pour chaque ou quelques cycles, tels que, par exemple, saturations d'oxygène coronaires veineux et artériels et leur différence et/ou un coefficient d'oxydo-réduction local artério-veineux, etc., pouvant alerter préventivement avant tout hypoxie d'une surconsommation d'oxygène du myocarde qui se manifeste par exemple d'abord par une accumulation extra-membranaire d'électrons.

Le dispositif de la stimulation STIO peut être associé à toutes les catégories de stimulateurs cardiaques automatiques DIA implantables, et aussi, notamment, les défibrillateurs automatiques, anti-tachycardiques, VVI, DDD, DDDR, etc.

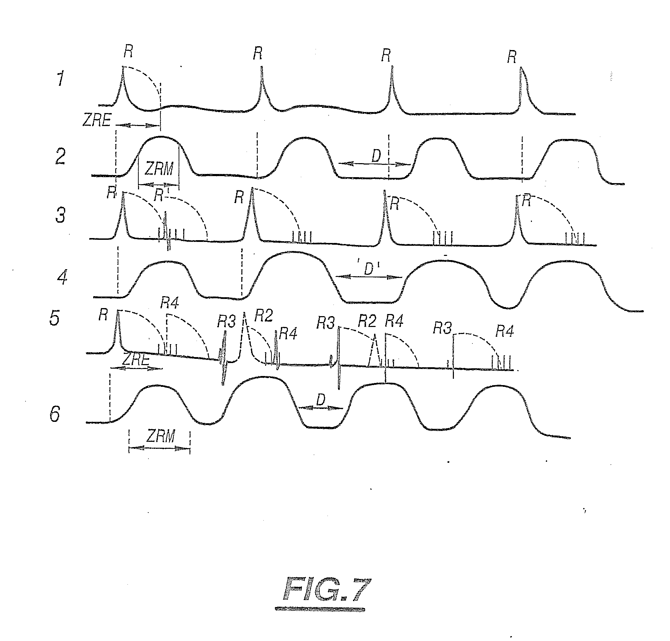

A titre d'exemple, on se réfère aux figures 7 à 9.

Dans cet exemple, le praticien ou le dispositif selon l'invention constate qu'un patient, souffrant d'une insuffisance cardiaque importante, possède un rythme cardiaque spontanée non accéléré, par exemple, inférieur à 125 p/mn, et par exemple préférentiellement inférieur à 100 p/mn.

La ligne 1 de la figure 7 montre le rythme régulier des ondes R (des complexes QRS) de ce patient, ZRE représentant la zone réfractaire électrique, déterminée, par exemple, par l'un des moyens décrits dans la présente invention.

La ligne 2 représente le mécanogramme détecté en réponse. Sur cette courbe on a aussi représenté, la période réfractaire mécanique ZRM, ainsi que la diastole mécanique D.

Sur la ligne 3, on a représenté la simulation électrocardiographique d'une stimulation isotrope selon l'invention, dans laquelle on envoie une salve couplée immédiatement, au moment de la fin de la zone réfractaire électrique ZRE et l'on voit le prolongement de la zone réfractaire qui suit le complexe R' provoqué par la stimulation couplée simulée. Dans cette simulation, les moyens de traitements informatiques du dispositif ont prévu que le rythme de base n'est guère modifié par la stimulation inotrope simulée et les complexes spontanés R surviennent donc comme ils le feraient en l'absence de stimulation.

Sur la ligne 4, on a représenté l'accroissement estimé, en durée et en intensité du mécanogramme, accroissement qui se constate immédiatement à partir de la deuxième onde R spontanée, c'est-à-dire la première qui suit la stimulation inotrope R'.

Il en résulte d'ailleurs une diminution de la durée diastolique mécanique D', qui est inférieure à D.

On procède alors à la stimulation inotrope réelle du coeur tel que prévue sur la courbe de simulation 2 de la figure 7, et l'on vérifie que l'on obtient immédiatement une augmentation de la performance hémodynamique dès le premier cycle consécutif, comme sur la ligne 4. On continue à faire cette vérification pendant une certaine durée ou un certain nombre de cycles cardiaques, par exemple correspondant à une ou plusieurs dizaines ou une centaine de cycles, étant entendu que l'on devrait normalement obtenir, après l'augmentation immédiate de la performance au second cycle, une augmentation progressive, supplémentaire s'étendant sur des dizaines ou centaines de cycles consécutifs.

On peut également simuler chez le même patient, l'effet qu'obtiendrait la stimulation inotrope selon l'invention, basée sur le rythme plus rapide imposé par des impulsions de stimulation R3 qui empêchent la survenue des signaux spontanées R (on a représenté par R2 les complexes QRS supprimés). On voit sur la ligne 5 l'électrocardiogramme correspondant à cette simulation avec couplage de salves et capture électrique du coeur.

La ligne 6 représente l'accroissement estimé du mécanogramme par cette simulation. Grâce à des moyens de comparaison du dispositif, on peut alors comparer les effets des simulations suivant les lignes 2 et 6, et, par exemple, dans le cas où il serait estimé que l'accroissement de performance hémodynamique provenant d'une stimulation couplée simple selon la ligne 3 est insuffisant, on pourra proposer la mise en oeuvre d'une stimulation selon la ligne 5 si l'accroissement et le mode espéré est supérieur et permet d'obtenir un accroissement de performance cardiaque acceptable, par exemple un doublement par rapport au débit cardiaque spontané tel qu'estimé sur la ligne 2.

Une fois le choix de la simulation effectué, le dispositif procède effectivement à une stimulation inotrope selon la ligne 3 comme décrit précédemment ou selon la ligne 5, en fonction du choix effectué, et les moyens d'acquisition de l'hémodynamique et/ou la consommation d'oxygène permettent alors de vérifier l'existence et l'ampleur de l'accroissement de la performance hémodynamique et de la comparer à l'accroissement simulé.

En l'absence de l'accroissement de la performance hémodynamique, par exemple au cours des deux premiers cycles de stimulation couplée ou pairée, le dispositif arrête la stimulation inotrope.

Au contraire, si un accroissement hémodynamique est constaté, la stimulation se poursuit, de préférence sur plusieurs dizaines, ou une ou plusieurs centaines de cycles. On compare alors la performance hémodynamique obtenue à celle résultant de la simulation. Si la performance obtenue est comparable à la performance ciblée, par exemple dans une fourchette de 15 % de la performance ciblée, on continue la stimulation inotrope. Si l'accroissement de la performance obtenue dépasse largement l'augmentation simulée, le dispositif peut éventuellement diminuer la performance, par exemple en prenant en compte un index de stress cardiaque CSI et en comparant des index de performance CPI et de stress CSI, comme cela est décrit dans le brevet US 5213098 incorporé ici par référence, et on peut éventuellement n'effectuer la simulation inotrope que pour une proportion réduite de cycles. Si au contraire, la performance constatée est largement inférieure à celle estimée comme nécessaire et prévue par la simulation, le dispositif peut cesser la simulation inotrope.

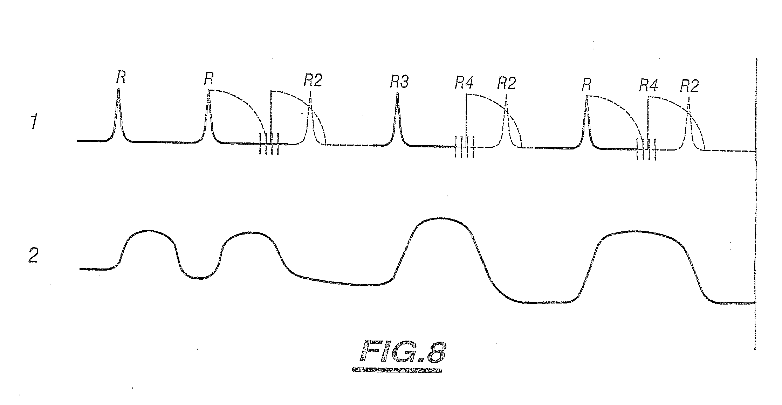

En se référant à la figure 8, on a représenté un cas d'insuffisance cardiaque associé à un rythme spontané tachycarde, par exemple supérieur à 125 cycle par minute. On a représenté le dernier cycle spontané R, ainsi que sur la ligne 2, la performance hémodynamique correspondante.

Dans un tel cas, le dispositif simule l'envoi d'une salve de stimulation vers la fin de la période réfractaire du dernier signal spontané R, ce qui prolonge la période réfractaire du coeur, de sorte que le complexe QRS suivant, qui se serait produit au moment R2 en raison de la tachycardie, ne peux plus se produire, ce qui provoque la survenue d'un complexe spontané R3 retardé. La salve de stimulation de couplage R4 est alors poursuivie et l'on voit qu'en empêchant chaque fois la survenue du complexe tachycardique spontané R2, on obtient, finalement, un rythme inférieur, par exemple divisé par 2.

Le plus souvent, cependant, on préfère assurer une prise en charge électrique complète du coeur ; le complexe spontané R3 est alors remplacé par une stimulation à un rythme un peu plus rapide.

A ce rythme normal stabilisé, correspond normalement une performance hémodynamique simulée accrue car l'effet RPE (PESP) surcompense largement la diminution du nombre de contractions par minute.

Si cette simulation permet d'espérer une performance hémodynamique suffisante, on met réellement en oeuvre la stimulation inotrope conformément à la procédure de la ligne 1 de la figure 8, et comme indiqué précédemment on vérifie la réponse et l'amplitude de la réponse obtenue pendant un, deux ou trois cycles puis pendant un nombre plus élevé de cycles. Dans une telle réalisation, on peut également, au cas où la performance hémodynamique simulée et/ou réellement obtenue ne serait pas suffisante, procéder à l'envoi d'une impulsion de stimulation à un rythme plus élevé.

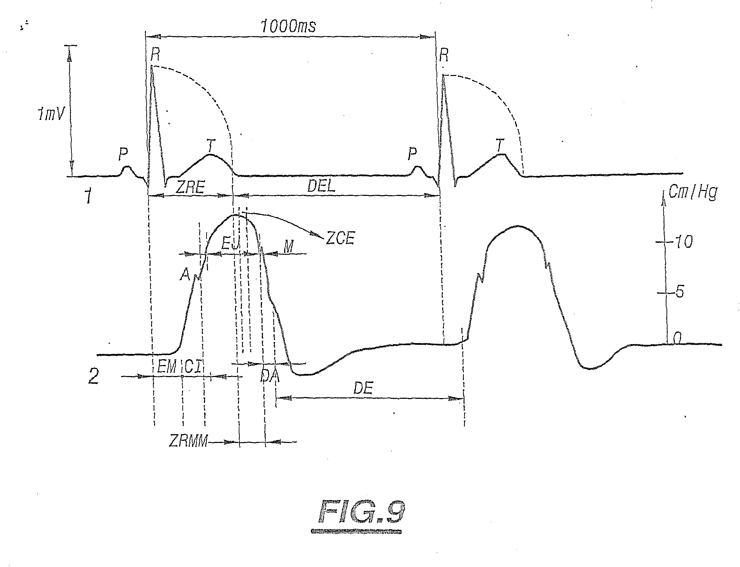

Sur la figure 9, on a représenté l'électrocardiogramme et le mécanogramme correspondant d'un patient. L'électrocardiogramme représenté sur la ligne 1 montre des complexes QRS et plus précisé-ment l'onde R spontanée ou stimulée à un rythme de base, par exemple de 60 pulsations/mn. L'apparition du complexe QRS crée une zone réfractaire électrique ZRE qui se termine plus ou moins avec l'onde T, sans qu'une corrélation suffisante puisse être généralement établie. Cette zone réfractaire est suivie de la diastole électriquement sensible DEL.

Sur la ligne 2, on voit que la montée en pression du mécanogramme s'effectue après un délai de couplage électromécanique EM, encore appelé E par la suite. Il se produit d'abord une contraction isovolumétrique tant que les valves ne sont pas ouvertes, pendant la durée CI. Cette contraction est suivie d'une contraction avec éjection sanguine EJ entre l'ouverture A de la valve aortique et l'ouverture M de la valve mitrale. DE représente la diastole mécanique, d'abord motrice, ensuite élastique et passive de remplissage.

La zone réfractaire mécanique maximale, pendant laquelle le muscle cardiaque est à l'état le plus contracté, est représentée par ZRMM. Cette zone, qui correspond au sommet de la courbe de pression, est, dans l'exemple représenté, relativement aplatie car la courbe de pression a été obtenue par un capteur mesurant la pression sanguine intracavitaire dans le ventricule. Cependant, lorsqu'on place un capteur de pression intramyocardique dans une région ou zone locale, la montée en pression locale est très nette et permet de bien distinguer facilement la zone réfractaire mécanique maximale locale proche de l'électrode active.

Dans une forme de réalisation de la présente invention, on acquiert l'électrocardiogramme de la ligne 1 dans cette région locale, par exemple de préférence la région myocardique adjacente à l'électrode ventriculaire de stimulation et détection, et l'on acquiert la courbe de pression intramyocardique également dans cette région myocardique. On raisonne dans la suite comme si les courbes de lignes 1 et 2 de la figure 9 représentaient ces courbes locales dans la région choisie.

On observe alors que la zone réfractaire électrique ZRE peut chevaucher partiellement ou même totalement la zone ZRMM. Lorsque la superposition est partielle, on définit, entre la fin de la zone ZRE et la fin de la zone ZRMM, une zone critique efficace ZCE à cibler, qui peut être égale à la zone ZRMM dans le cas où la zone réfractaire électrique ZRE se termine avant le début de la zone ZRMM, et qui peut être nulle si ZRE est allongée et englobe la durée de la zone ZRMM.

Conformément à la forme de réalisation de loin préférée de l'invention, la stimulation couplée ou pairée est envoyée pendant la zone ZCE. Celle-ci peut être assez souvent de l'ordre de 30 à 40 ms et, dans ce cas, l'envoi, suffisamment tôt vers la fin de ZRE, d'une salve d'impulsions ayant un intervalle entre les impulsions, par exemple de 20 ms, provoquera certainement une stimulation dans la zone ZCE ciblée, sans augmentation péjorative de la consommation d'oxygène locale, stimulation qui produira le renforcement post-extra-systolique attendu avec l'augmentation la plus faible de consommation d'oxygène, par rapport à l'augmentation du travail mécanique du myocarde RPE (PEPS).

Si la zone ZCE tend à se rétrécir, le dispositif pourra alors avantageusement diminuer l'intervalle entre les impulsions de la salve de façon à garantir qu'une impulsion de la salve tombera dans cette zone ZCE. Cependant, on préférera ne pas raccourcir la durée entre deux impulsions consécutives sensiblement au-dessous de 10 ms, les moyens électroniques ne permettant pas, dans ce cas, d'identifier l'impulsion stimulante de la salve.

En conséquence, on préfère, si la zone ZCE est inférieure à 10 ou 15 ms, ne pas envoyer d'impulsions pairées ou couplées.

Il en sera de même si l'on constate sur un certain nombre de cycles que la zone ZCE varie de façon trop anarchique en durée ou dans le temps.

Dans une forme de réalisation particulièrement avantageuse, on détectera la zone ZRE par l'utilisation de trains d'impulsions comme cela a été décrit plus avant, et on mémorisera les positions et les durées des zones ZCE d'une pluralité de cycles, par exemple de 30 à 3000 cycles, pour détecter si la zone ZCE est à peu près stable ou si elle présente une tendance vers la diminution ou l'augmentation et on pourra alors intervenir en conséquence, cette fois-ci en envoyant une impulsion de stimulation, de préférence plutôt vers la fin estimée de la zone ZRMM du cycle en cours, en faisant l'hypothèse que la zone ZCE sera existante à ce moment.

On pourra ainsi, grâce ces mémorisations et vérifications qui pourront être effectuées par le simulateur analyseur SIMA selon l'invention, faire en sorte que seul un faible nombre et même aucune impulsion assurant une dépolarisation électrique ne survienne en dehors de la zone ZRMM, permettant ainsi de limiter la consommation locale d'oxygène provoquée par la stimulation pairée ou couplée inotrope. En choisissant convenablement dans le coeur l'endroit où l'on détecte la pression intramyocardique et la dépolarisation électrique et en stimulant dans ou à proximité de cet endroit, on peut faire en sorte que la propagation intramyocardique de la dépolarisation, provoquée par la stimulation inotrope, se fasse en concordance avec la propagation persistante d'une contraction forte, et notamment d'une contraction myocardique maximale dans les nouveaux territoires où se propage la dépolarisation de stimulation, de sorte que la consommation d'oxygène résultant de la stimulation pairée ou couplée restera également limitée dans une grande partie, voire la totalité du myocarde ventriculaire. Pour cela, on préfèrera détecter la zone ZRMM dans une zone myocardique initiale où débute la propagation de la dépolarisation, l'influx électrique se propageant alors dans le reste du myocarde en même temps et à vitesse sensiblement égale que la contraction maximale, leur couplage restant suffisamment constant.

Dans certains cas particuliers, par exemple si l'on a pu détecter une modification du fonctionnement des faisceaux intramyocardiques de conduction, par exemple en cas de blocs de branche ou de zones locales pathologiques, on pourra détecter la zone ZRMM et éventuellement les signaux de dépolarisation électriques correspondants dans plusieurs endroits et stimuler dans ces endroits pendant leurs zones ZRMM ou plus précisément ZCE respectives.

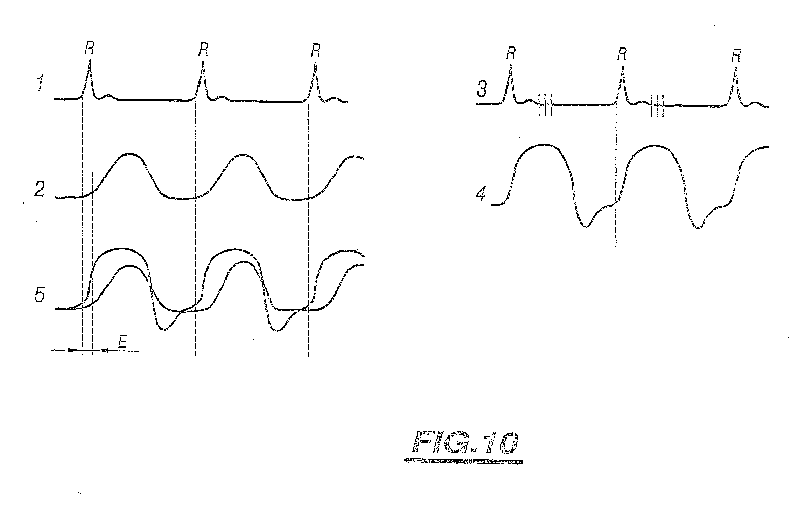

Sur la figure 10, on a représenté l'accroissement de l'efficacité hémodynamique simulé sur trois cycles spontanés chez un patient.

La ligne 1 représente le rythme spontané par exemple de 90 p/mm chez un insuffisant cardiaque aigu.

La ligne 2 représente la variation de pression spontanée dans le ventricule gauche généré par le rythme spontané.

La ligne 3 représente l'électrogramme d'une stimulation pairée simulée chez ce même patient, qui maintient un rythme identique ou similaire à celui de la ligne 1.

La ligne 4 représente la variation simulée de pression intra-ventriculaire gauche qui en résulte.

La ligne 5 consiste en une superposition des différences de pression réelles de la ligne 2 et simulées de la ligne 4, les différences étant hachurées. On voit notamment que la pente initiale de montée en pression Δp/Δt systémique augmente de 25 % et la pente Δp/Δt diastolique, c'est-à-dire de diminution de pression de plus 30 %.

Le débit par systole est augmenté de 35 % et le débit par minute de 33 %.

La durée E de couplage électromécanique est réduite de 30 %.

L'analyse de cette simulation montre une augmentation significative de l'hémodynamique.

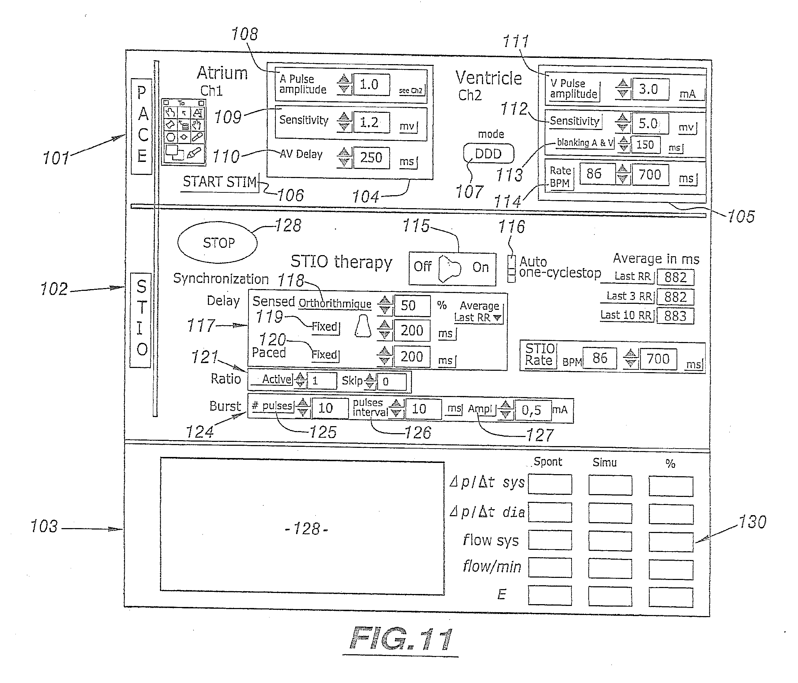

Sur la figure 11, on a représenté le panneau de commande d'un dispositif SIMA selon l'invention, divisé en trois parties 101, 102, 103.

Le panneau 101 comporte un sous-panneau de commande auriculaire 104 correspondant à des électrodes de stimulation et de détection auriculaire et un sous-panneau ventriculaire 105 correspondant à des électrodes de stimulation et de détection ventriculaire. Le panneau 104 présente trois boutons de réglage, à savoir 108 pour choisir l'amplitude des impulsions de stimulation, 109 pour la sensibilité de détection et 110 pour la durée entre les stimulations auriculaires et ventriculaires si l'on choisit le mode DDD. Le panneau ventriculaire 105 comporte trois boutons 111, 112, 113 pour le réglage et l'amplitude des impulsions ventriculaires, de la sensibilité de l'électrode de détection ventriculaire et du décalage auriculo-ventriculaire. Le bouton 114 permet de définir le rythme de stimulation orthorythmique. Le bouton 106 permet de mettre en oeuvre ou d'interrompre la stimulation et le bouton 107 de passer en mode auriculaire, ventriculaire ou DDD.

Le panneau 102 comporte les boutons de commande de stimulation-STIO selon l'invention. Le bouton 115 permet de mettre en oeuvre ou d'interrompre la stimulation STIO et le bouton 116 permet de ne déclencher une stimulation automatique que pour un seul cycle.

Le sous-panneau 117 présente un ensemble de boutons, à savoir 118 pour le couplage (en pourcentage), 119 pour le couplage en ms. fixe, 120 pour afficher la période de la stimulation de base.

Le sous-panneau 121 permet de choisir la proportion de cycles à stimuler.

Le sous-panneau 124 présente trois boutons 125, 126 et 127 permettant de définir les paramètres des impulsions de la salve d'impulsions de stimulation inotrope, à savoir le nombre d'impulsions dans la salve par le bouton 125, l'intervalle entre les deux impulsions consécutives dans la salve par les boutons 126, et l'amplitude de l'impulsion de la salve (bouton 127).

Le bouton d'arrêt d'urgence 128 permet l'arrêt immédiat de la stimulation inotrope.

La partie inférieure 103 permet d'afficher et de régler la simulation SIMA selon l'invention, et de comparer la simulation aussi bien aux paramètre détectés initialement en actionnant éventuellement la partie 101, qu'aux paramètres provenant de l'actionnement d'une STIO par le tableau 102.

La partie 103 comporte un écran 128 sur lequel apparaissent les courbes électriques, mécaniques réelles et simulées et leur juxtaposition, comme représenté sur la fig. 10.

Un sous-panneau 130 affiche les valeurs spontanées détectées (spont), simulées correspondantes (simu), et leur comparaisons en pourcentage de valeur spontanée, pour les paramètres Δp/Δt diastolique, débit ventriculaire systolique, débit ventriculaire par minute et valeur E du couplage électromécanique du ventricule.

## Revendications

1. Procédé de stimulation *in vitro* de cellules destinées à être implantées dans le coeur, ledit procédé comprenant les étapes suivantes :
- obtenir et cultiver, *in vitro*, des cellules de régénération, de préférence sous forme de petits amas, ou de cultures confluentes, pour être en contact entre elles ;
- mettre ces cellules confluentes en contact de conduction électrique entre elles et éventuellement avec les cellules d'un tissu myocardique préalablement prélevé et avec un dispositif de stimulation électrique,
- adresser périodiquement des impulsions électriques auxdites cellules cultivées ;
- détecter les réponses de dépolarisation électrique des cellules et/ou potentiels de membrane, et/ou zones réfractaires électriques.

2. Procédé selon la revendication 1, dans laquelle la stimulation est une stimulation électrique simple à un rythme, de préférence, de l'ordre d'un rythme cardiaque normal, suivie après une période initiale, d'une stimulation pairée ou couplée selon l'une des revendications 1 à 20, une fois que les amas ou groupes de cellules en culture sont synchronisés pour manifester une période réfractaire électrique notamment une période relativement proche de celle des cellules du coeur destiné à recevoir les cellules.

3. Procédé selon l'une des revendications 1 et 2, dans laquelle les cellules ont été modifiées pour surexprimer la télomérase ou protéine Sir2.

4. Procédé de préparation de cellules vivantes, notamment végétales, animales et humaines, susceptibles d'être réimplantées à titre prophylactique ou thérapeutique, dans lequel on réalise le transport dans un ovocyte, de préférence non fécondé ou récemment fécondé, de mammifère homologue ou hétérologue, préalablement de préférence complètement ou partiellement vidé de son noyau, d'un noyau d'une cellule dédifférenciée, de façon à induire un stade de mitose dudit noyau transféré, en ce que l'on prélève ce noyau pendant la mitose et avant la fin de celle-ci, puis l'on introduit ce noyau partiellement dédifférencié en mitose à ce stade dans une cellule, de préférence après en avoir retiré une partie ou la totalité du ou de ses noyau(x), de façon à induire et à terminer sa division nucléaire de différenciation et former une souche cellulaire ou un tissu à un stade de différenciation moins avancé que celui de ladite cellule différenciée.

5. Procédé selon l'une des revendications 1 à 4, **caractérisé en ce que** l'on extrait le noyau transféré au stade de la métaphase de la première mitose.

6. Procédé selon l'une des revendications 1 à 4, **caractérisé en ce que** l'on extrait le noyau transféré au stade de l'anaphase de la première mitose.

7. Procédé selon l'une des revendications 1 à 4, **caractérisé en ce que** l'on retire le noyau transféré au stade de la prophase de la première mitose.

8. Procédé selon l'une des revendications 1 à 4, **caractérisé** selon que l'on retire le noyau transféré au stade de la télophase de la première mitose.

9. Procédé selon l'une des revendications 1 à 8, **caractérisé en ce que** l'on transfère dans l'ovocyte, un noyau de cellule myocardique, musculaire ou autorythmique cardiaque, notamment cellules sinusales, du centre de Tawara ou fibres du faisceau de His ou de Purkinje.

10. Procédé selon la revendication 9, **caractérisé en ce que** l'on introduit le noyau prélevé avant la fin de sa mitose intra-ovocytaire dans une cellule myocardique ou musculaire, de préférence vidée de son noyau ou d'une partie ou de la totalité de ses noyaux.

11. Procédé selon l'une des revendications 9 et 10, **caractérisé en ce que** l'on soumet les cellules partiellement dédifférenciées obtenues, de préférence après ou pendant leur culture de multiplication cellulaire après la formation d'un ensemble confluent, à une stimulation périodique électrique du type stimulation cardiaque.

12. Procédé selon la revendication 11, **caractérisé en ce que** l'on soumet lesdites cellules à une stimulation électrique couplée ou pairée dans laquelle cette impulsion sans aucun effet contractile est adressée peu après la fin de la période réfractaire électrique des cellules en culture.

13. Procédé selon la revendication 11, **caractérisé en ce que** l'on soumet lesdites cellules à une stimulation électrique selon des cycles comprenant une première impulsion de stimulation et, vers la fin de la période réfractaire, un train d'impulsions, de façon que l'une au moins des impulsions du train tombe peu après la fin de la période réfractaire électrique des cellules et durant leur zone réfractaire mécanique de contraction maximale.

14. Procédé dans lequel on implante les cellules obtenues par le procédé selon l'une des revendications 1 à 13 au niveau du myocarde auriculaire, notamment dans le cas d'un patient souffrant d'une fibrillation auriculaire.

15. Procédé selon l'une des revendications 4 à 8, dans lequel on transfère dans l'ovocyte des noyaux, ou parties, de cellules de follicules pileux et/ou de mélanocytes permettant une régénération de chevelure et/ou de sa coloration.

16. Segment ou stent artériel, notamment à visée coronaire, aortique, carotide, rénale ou fémorale, comprenant une structure revêtue ou colonisée par des cellules obtenues par le procédé selon l'une des revendications 1 à 13.

17. Segment ou stent artériel physiologique constitué de cellules vivantes, auto-contractiles et élastiques, notamment autologues, cultivées selon l'une des revendications 1 à 13.

18. Segment ou stent artériel selon la revendication 16 ou 17, conformé à la façon d'un stent artériel agencé pour être introduit dans une lumière artérielle.

19. Segment ou stent selon la revendication 16 à 18, comprenant des moyens assurant une stimulation électrique de type artériel, coordonnée avec la diastole ventriculaire, desdites cellules du segment.

20. Segment ou stent selon l'une des revendications 16 à 19, comprenant au moins une électrode de stimulation et/ou de détection.

21. Segment ou stent selon la revendication 20, dans laquelle l'électrode de stimulation et/ou de détection a été introduite dans la culture cellulaire pour être entourée par lesdites cellules vivantes.

22. Segment ou stent selon l'une des revendications 16 à 21 comprenant un capteur, notamment de mesure de saturation d'oxygène et/ou de paramètres métaboliques, et un capteur électrocardiographique de détection ou stimulation.

23. Segment ou stent selon l'une des revendications 16 à 22 comprenant une pluralité d'électrodes agencées pour obtenir les variations de l'impédance électrique locale.

24. Segment artériel ou stent selon l'une des revendications 16 à 23 présentant une structure, notamment en mailles expansibles supportant des couches cellulaires différentes, telles qu'endoartère, myoartère et périartère, ladite structure permettant une croissance spontanée avec élargissement progressif de sa lumière et création d'une vascularisation nourrissant notamment la partie myoartérielle.

25. Segment selon l'une des revendications 16 à 24, **caractérisé en ce que** la structure est réalisée en au moins l'un des matériaux suivants : PLGA, collagène, globine.

26. Stent artériel sans cellules vivantes possédant une structure radialement expansible, mais assez rigide pour maintenir la lumière artérielle ouverte au moins en systole, éventuellement biodégradable ou retirable par cathéter et susceptible de recevoir un stent physiologique selon l'une des revendications 16 à 25.

27. Bloc tissulaire destiné à être implanté dans ou sur le coeur et comprenant un segment selon l'une des revendications 16 à 25, entouré de son bloc fonctionnel de tissu vasculo-myocardique périphérique pourvu d'artères, d'artérioles et réseau capillaire et veineux, pouvant être cousu ou ancré dans ou sur le myocarde du receveur.

28. Pacemaker cardiaque biologique comprenant des cellules ou tissus autorythmiques cardiaques, partiellement dédifférenciées, selon l'une des revendications 1 à 13, de préférence autologue, homologue, provenant de l'organisme receveur, et destiné à être implanté dans le coeur ou une région défaillante du coeur.
